# EUROPEAN PATENT APPLICATION

(11) **EP 1 188 828 A1**
(43) Date of publication of application: **20.03.2002**
(21) Application number: 00935616.3
(22) Date of filing: 09.06.2000
(51) Int. Cl.: C12N 15/09, C07K 14/435, C07K 16/18, C12N 1/15, C12N 1/21, C12N 5/10, C12P 21/02, G01N 33/15, G01N 33/50, G01N 33/566, A61K 31/00, A61K 38/00, A61K 45/00

(54) **REG-BINDING PROTEIN**

(30) Priority: 10.06.1999 JP 16448899
(71) Applicant: Okamoto, Hiroshi, Sendai-shi, Miyagi 980-0874 (JP)
(72) Inventor: Okamoto, Hiroshi, Sendai-shi, Miyagi 980-0874 (JP)
(74) Representative: VOSSIUS & PARTNER
(86) International application number: JP0003764
(87) International publication number: WO0077192

(57) **Abstract**

The inventors succeeded in cloning a protein binding to Reg protein from rat pancreatic Langerhans' islet-derived cDNA. It was revealed that the protein expressed on the COS cell surface specifically binds to Reg protein. When the Reg-binding protein is expressed in RINm5F β cells, DNA synthesis and cell proliferation are stimulated depending on the dose of the Reg protein added to the medium, and at a higher concentration, apoptosis is induced. It is considered that the protein functions as a Reg receptor expressed on the surface of cells such as β cells and regulates the proliferation of these cells. The protein and gene thereof are useful in developing novel therapeutic agents for diabetes.

SEQUENCE LISTING

<110> OKAMOTO, Hiroshi

<120> Reg-binding protein

<130> OKT-101PCT

<140>
<141>

<150> JP 1999-164488
<151> 1999-06-10

<160> 9

<170> PatentIn Ver. 2.0

<210> 1
<211> 1599
<212> DNA
<213> Rattus norvegicus

<220>
<221> CDS
<222> (168)..(1259)

<400> 1

tcagcgagga aaatgaaatt cccattttat ttggtgcctt gtgcagggag cacactgatc

60

cctctagaac cttgtgtgtg aaaaagaggt cgagttttgt caaacagact catggttatg

120

gcaagtgatc cgacgtgacc agagtgggca agagccacag tgaactc atg aca ggc

176

Met Thr Gly

1

tat acc atg ttg cgg aat ggg gga gtg ggg aac ggt ggt cag acc tgt 224

Tyr Thr Met Leu Arg Asn Gly Gly Val Gly Asn Gly Gly Gln Thr Cys

    5                   10                  15

atg ctg cgc tgg tcc aac cgc atc cgg ctg acc tgg ctg agt ttc acg 272

Met Leu Arg Trp Ser Asn Arg Ile Arg Leu Thr Trp Leu Ser Phe Thr

20                  25                  30                  35

ctg ttc atc atc ctg gtc ttc ttc ccc ctc att gcc cac tat tac ctc 320

Leu Phe Ile Ile Leu Val Phe Phe Pro Leu Ile Ala His Tyr Tyr Leu

                40                  45                  50

acc act ctg gat gag gca gat gag gcc ggc aag cgc atc ttt ggc ccc 368

Thr Thr Leu Asp Glu Ala Asp Glu Ala Gly Lys Arg Ile Phe Gly Pro
            55                  60                  65

cgg gct ggc aac gag ctc tgt gag gta aag cac gtc cta gat ctt tgt 416
Arg Ala Gly Asn Glu Leu Cys Glu Val Lys His Val Leu Asp Leu Cys
        70                  75                  80

cgg atc cgc gag tct gtg agc gaa gag ctt cta cag cta gaa gcc aag 464
Arg Ile Arg Glu Ser Val Ser Glu Glu Leu Leu Gln Leu Glu Ala Lys
    85                  90                  95

cgg cag gag ctg aac agc gag att gcc aag cta aac ctc aag att gaa 512
Arg Gln Glu Leu Asn Ser Glu Ile Ala Lys Leu Asn Leu Lys Ile Glu
100                 105                 110                 115

gcc tgt aag aag agt ata gag aac gcc aag cag gac ctg ctg cag ctc 560
Ala Cys Lys Lys Ser Ile Glu Asn Ala Lys Gln Asp Leu Leu Gln Leu
                120                 125                 130

aag aat gtc att agc cag aca gag cac tcc tac aag gag ctg atg gcc 608
Lys Asn Val Ile Ser Gln Thr Glu His Ser Tyr Lys Glu Leu Met Ala
            135                 140                 145

cag aac cag ccc aaa ctg tca ctg ccc atc cgg ctg ctc cct gag aag 656
Gln Asn Gln Pro Lys Leu Ser Leu Pro Ile Arg Leu Leu Pro Glu Lys
        150                 155                 160

gat gac gct ggc ctt cca ccc ccc aag gtc act cgg ggt tgc cgg cta 704
Asp Asp Ala Gly Leu Pro Pro Pro Lys Val Thr Arg Gly Cys Arg Leu
    165                 170                 175

cac aac tgc ttc gat tac tct cgt tgc cct ctg acg tct ggc ttt cct 752
His Asn Cys Phe Asp Tyr Ser Arg Cys Pro Leu Thr Ser Gly Phe Pro
180                 185                 190                 195

gtc ttc gtc tat gac agt gac cag ttt gcc ttt ggg agc tac ctg gac 800
Val Phe Val Tyr Asp Ser Asp Gln Phe Ala Phe Gly Ser Tyr Leu Asp
                200                 205                 210

cct ttg gtc aag cag gct ttt cag gct aca gtg aga gcc aac gtt tat 848
Pro Leu Val Lys Gln Ala Phe Gln Ala Thr Val Arg Ala Asn Val Tyr
            215                 220                 225

gtt aca gaa aat gca gcc atc gcc tgc ctg tat gtg gtg tta gtg gga 896
Val Thr Glu Asn Ala Ala Ile Ala Cys Leu Tyr Val Val Leu Val Gly
        230                 235                 240

gag ata caa gag ccc gct gtg ctg cag cct gcc gac ctt gag aag cag 944
Glu Ile Gln Glu Pro Ala Val Leu Gln Pro Ala Asp Leu Glu Lys Gln
    245                 250                 255

ctg cat tct ctg cca cac tgg agg aca gac gga cac aac cat gtc atc 992

Leu His Ser Leu Pro His Trp Arg Thr Asp Gly His Asn His Val Ile
260                 265                 270                 275

atc aat ctg tcc cgg aag tca gac aca caa aat tta ctg tac aat gtc 1040
Ile Asn Leu Ser Arg Lys Ser Asp Thr Gln Asn Leu Leu Tyr Asn Val
                280                 285                 290

agt aca ggt cgg gcc atg gtg gcc cag tct acc ttc tat gct gcc cag 1088
Ser Thr Gly Arg Ala Met Val Ala Gln Ser Thr Phe Tyr Ala Ala Gln
            295                 300                 305

tac aga gct ggc ttt gac ttg gtt gtg tca cca ctt gtc cat gcc atg 1136
Tyr Arg Ala Gly Phe Asp Leu Val Val Ser Pro Leu Val His Ala Met
        310                 315                 320

tct gaa ccc aac ttc atg gaa atc cca cgt gta act att ttt tca ctt 1184
Ser Glu Pro Asn Phe Met Glu Ile Pro Arg Val Thr Ile Phe Ser Leu
    325                 330                 335

ggg aga ggt gag gaa gaa caa gag aag ctg ggg gtg tgg aga ggc aga 1232
Gly Arg Gly Glu Glu Glu Gln Glu Lys Leu Gly Val Trp Arg Gly Arg
340                 345                 350                 355

ccc ccc cca ggc tgg ggt gct ggc ccc tagactaggg tgctgacccc
1279
Pro Pro Pro Gly Trp Gly Ala Gly Pro

                360

tgggctgggg tgctgcgtgc tacctcccac tgtgaaatcg atggtgctca caattgtctc

1339

ttgtaatgta tgtgattttt ttttaaggag aaaaagaaac tatttaagat tctgaaggtg

1399

ctactatttt tgttgccaca ggctttaaag aaactttctg agtgggtggg gccttgccca

1459

cttatctttc tctcctccaa atgaggagtt aaaaatgtta ctaaattgcc cgcacgtgta

1519

atccgctgaa aagaaaaaaa aaaaagaaaa aaaaaaggaa ggaaagaagg aaagaaggaa

1579

ggaaggaagg aaggaaagga

1599

<210> 2

<211> 364

<212> PRT

<213> Rattus norvegicus

<400> 2

Met Thr Gly Tyr Thr Met Leu Arg Asn Gly Gly Val Gly Asn Gly Gly

1               5                   10                  15

Gln Thr Cys Met Leu Arg Trp Ser Asn Arg Ile Arg Leu Thr Trp Leu

            20                  25                  30

Ser Phe Thr Leu Phe Ile Ile Leu Val Phe Phe Pro Leu Ile Ala His

        35                  40                  45

Tyr Tyr Leu Thr Thr Leu Asp Glu Ala Asp Glu Ala Gly Lys Arg Ile

    50                  55                  60

Phe Gly Pro Arg Ala Gly Asn Glu Leu Cys Glu Val Lys His Val Leu

65                  70                  75                  80

Asp Leu Cys Arg Ile Arg Glu Ser Val Ser Glu Glu Leu Leu Gln Leu

                85                  90                  95

Glu Ala Lys Arg Gln Glu Leu Asn Ser Glu Ile Ala Lys Leu Asn Leu

            100                 105                 110

Lys Ile Glu Ala Cys Lys Lys Ser Ile Glu Asn Ala Lys Gln Asp Leu

        115                 120                 125

Leu Gln Leu Lys Asn Val Ile Ser Gln Thr Glu His Ser Tyr Lys Glu

    130                 135                 140

Leu Met Ala Gln Asn Gln Pro Lys Leu Ser Leu Pro Ile Arg Leu Leu

145                 150                 155                 160

Pro Glu Lys Asp Asp Ala Gly Leu Pro Pro Pro Lys Val Thr Arg Gly

                165                 170                 175

Cys Arg Leu His Asn Cys Phe Asp Tyr Ser Arg Cys Pro Leu Thr Ser

            180                 185                 190

Gly Phe Pro Val Phe Val Tyr Asp Ser Asp Gln Phe Ala Phe Gly Ser

        195                 200                 205

Tyr Leu Asp Pro Leu Val Lys Gln Ala Phe Gln Ala Thr Val Arg Ala

    210                 215                 220

Asn Val Tyr Val Thr Glu Asn Ala Ala Ile Ala Cys Leu Tyr Val Val

225                 230                 235                 240

Leu Val Gly Glu Ile Gln Glu Pro Ala Val Leu Gln Pro Ala Asp Leu

                245                 250                 255

Glu Lys Gln Leu His Ser Leu Pro His Trp Arg Thr Asp Gly His Asn

            260                 265                 270

His Val Ile Ile Asn Leu Ser Arg Lys Ser Asp Thr Gln Asn Leu Leu
        275                 280                 285

Tyr Asn Val Ser Thr Gly Arg Ala Met Val Ala Gln Ser Thr Phe Tyr
    290                 295                 300

Ala Ala Gln Tyr Arg Ala Gly Phe Asp Leu Val Val Ser Pro Leu Val
305                 310                 315                 320

His Ala Met Ser Glu Pro Asn Phe Met Glu Ile Pro Arg Val Thr Ile
                325                 330                 335

Phe Ser Leu Gly Arg Gly Glu Glu Glu Gln Glu Lys Leu Gly Val Trp
            340                 345                 350

Arg Gly Arg Pro Pro Pro Gly Trp Gly Ala Gly Pro
        355                 360

<210> 3
<211> 3198
<212> DNA
<213> Rattus norvegicus

<220>
<221> CDS

<222> (5)..(2761)

<400> 3

actc atg aca ggc tat acc atg ttg cgg aat ggg gga gtg ggg aac ggt 49
Met Thr Gly Tyr Thr Met Leu Arg Asn Gly Gly Val Gly Asn Gly
1               5                   10                  15

ggt cag acc tgt atg ctg cgc tgg tcc aac cgc atc cgg ctg acc tgg 97
Gly Gln Thr Cys Met Leu Arg Trp Ser Asn Arg Ile Arg Leu Thr Trp
                20                  25                  30

ctg agt ttc acg ctg ttc atc atc ctg gtc ttc ttc ccc ctc att gcc 145
Leu Ser Phe Thr Leu Phe Ile Ile Leu Val Phe Phe Pro Leu Ile Ala
            35                  40                  45

cac tat tac ctc acc act ctg gat gag gca gat gag gcc ggc aag cgc 193
His Tyr Tyr Leu Thr Thr Leu Asp Glu Ala Asp Glu Ala Gly Lys Arg
        50                  55                  60

atc ttt ggc ccc cgg gct ggc aac gag ctc tgt gag gta aag cac gtc 241
Ile Phe Gly Pro Arg Ala Gly Asn Glu Leu Cys Glu Val Lys His Val
    65                  70                  75

cta gat ctt tgt cgg atc cgc gag tct gtg agc gaa gag ctt cta cag 289
Leu Asp Leu Cys Arg Ile Arg Glu Ser Val Ser Glu Glu Leu Leu Gln
80                  85                  90                  95

cta gaa gcc aag cgg cag gag ctg aac agc gag att gcc aag cta aac 337
Leu Glu Ala Lys Arg Gln Glu Leu Asn Ser Glu Ile Ala Lys Leu Asn
                100                 105                 110
ctc aag att gaa gcc tgt aag aag agt ata gag aac gcc aag cag gac 385
Leu Lys Ile Glu Ala Cys Lys Lys Ser Ile Glu Asn Ala Lys Gln Asp
            115                 120                 125
ctg ctg cag ctc aag aat gtc att agc cag aca gag cac tcc tac aag 433
Leu Leu Gln Leu Lys Asn Val Ile Ser Gln Thr Glu His Ser Tyr Lys
        130                 135                 140
gag ctg atg gcc cag aac cag ccc aaa ctg tca ctg ccc atc cgg ctg 481
Glu Leu Met Ala Gln Asn Gln Pro Lys Leu Ser Leu Pro Ile Arg Leu
    145                 150                 155
ctc cct gag aag gat gac gct ggc ctt cca ccc ccc aag gtc act cgg 529
Leu Pro Glu Lys Asp Asp Ala Gly Leu Pro Pro Pro Lys Val Thr Arg
160                 165                 170                 175
ggt tgc cgg cta cac aac tgc ttc gat tac tct cgt tgc cct ctg acg 577
Gly Cys Arg Leu His Asn Cys Phe Asp Tyr Ser Arg Cys Pro Leu Thr
                180                 185                 190
tct ggc ttt cct gtc ttc gtc tat gac agt gac cag ttt gcc ttt ggg 625

Ser Gly Phe Pro Val Phe Val Tyr Asp Ser Asp Gln Phe Ala Phe Gly
            195                 200                 205

agc tac ctg gac cct ttg gtc aag cag gct ttt cag gct aca gtg aga 673
Ser Tyr Leu Asp Pro Leu Val Lys Gln Ala Phe Gln Ala Thr Val Arg
        210                 215                 220

gcc aac gtt tat gtt aca gaa aat gca gcc atc gcc tgc ctg tat gtg 721
Ala Asn Val Tyr Val Thr Glu Asn Ala Ala Ile Ala Cys Leu Tyr Val
    225                 230                 235

gtg tta gtg gga gag ata caa gag ccc gct gtg ctg cag cct gcc gac 769
Val Leu Val Gly Glu Ile Gln Glu Pro Ala Val Leu Gln Pro Ala Asp
240                 245                 250                 255

ctt gag aag cag ctg cat tct ctg cca cac tgg agg aca gac gga cac 817
Leu Glu Lys Gln Leu His Ser Leu Pro His Trp Arg Thr Asp Gly His
                260                 265                 270

aac cat gtc atc atc aat ctg tcc cgg aag tca gac aca caa aat tta 865
Asn His Val Ile Ile Asn Leu Ser Arg Lys Ser Asp Thr Gln Asn Leu
            275                 280                 285

ctg tac aat gtc agt aca ggt cgg gcc atg gtg gcc cag tct acc ttc 913
Leu Tyr Asn Val Ser Thr Gly Arg Ala Met Val Ala Gln Ser Thr Phe
        290                 295                 300

tat gct gcc cag tac aga gct ggc ttt gac ttg gtt gtg tca cca ctt 961
Tyr Ala Ala Gln Tyr Arg Ala Gly Phe Asp Leu Val Val Ser Pro Leu
    305                 310                 315

gtc cat gcc atg tct gaa ccc aac ttc atg gaa atc cca ccg cag gtg 1009
Val His Ala Met Ser Glu Pro Asn Phe Met Glu Ile Pro Pro Gln Val
320                 325                 330                 335

cca gtt aag cgg aaa tat ctc ttc act ttc cag ggt gag aag att gag 1057
Pro Val Lys Arg Lys Tyr Leu Phe Thr Phe Gln Gly Glu Lys Ile Glu
                340                 345                 350

tct cta aga tct agc ctt cag gag gcc cgt tcc ttt gag gaa gaa atg 1105
Ser Leu Arg Ser Ser Leu Gln Glu Ala Arg Ser Phe Glu Glu Glu Met
            355                 360                 365

gag ggt gac cct ccg gcc gac tat gat gat cga atc att gcc acc ctc 1153
Glu Gly Asp Pro Pro Ala Asp Tyr Asp Asp Arg Ile Ile Ala Thr Leu
        370                 375                 380

aag gcc gta cag gac agc aag cta gat cag gtg ctg gta gaa ttt act 1201
Lys Ala Val Gln Asp Ser Lys Leu Asp Gln Val Leu Val Glu Phe Thr
    385                 390                 395

tgc aaa aac cag cca aag ccc agt ctg cct act gag tgg gca ctg tgt 1249

Cys Lys Asn Gln Pro Lys Pro Ser Leu Pro Thr Glu Trp Ala Leu Cys
400                 405                 410                 415

ggg gag cgg gag gac cgg cta gag tta ctg aag ctc tcc acc ttc gcc     1297
Gly Glu Arg Glu Asp Arg Leu Glu Leu Leu Lys Leu Ser Thr Phe Ala
                420                 425                 430

ctc atc atc act ccc ggg gac ccg agc ctg ctt atc tca tct ggc tgt     1345
Leu Ile Ile Thr Pro Gly Asp Pro Ser Leu Leu Ile Ser Ser Gly Cys
            435                 440                 445

gca aca cgg ctc ttt gaa gcc ttg gag gtg gga gct gtg cct gtt gtc     1393
Ala Thr Arg Leu Phe Glu Ala Leu Glu Val Gly Ala Val Pro Val Val
        450                 455                 460

ctt ggg gag cag gtg cag ctt ccg tac cac gac atg cta caa tgg aat     1441
Leu Gly Glu Gln Val Gln Leu Pro Tyr His Asp Met Leu Gln Trp Asn
    465                 470                 475

gag gcc gcc cta gtg gtg ccc aag cct cgt gtt aca gag gtt cac ttc     1489
Glu Ala Ala Leu Val Val Pro Lys Pro Arg Val Thr Glu Val His Phe
480                 485                 490                 495

ctg tta cga agt ctg tca gac agt gat ctg ttg gct atg agg cgg caa     1537
Leu Leu Arg Ser Leu Ser Asp Ser Asp Leu Leu Ala Met Arg Arg Gln
                500                 505                 510

ggc cgc ttt ctc tgg gag acc tac ttc tcc acc gct gac agt att ttt 1585
Gly Arg Phe Leu Trp Glu Thr Tyr Phe Ser Thr Ala Asp Ser Ile Phe
            515                 520                 525

aat acc gtg ctg gcc atg att agg act cga att cag atc cca gct gct 1633
Asn Thr Val Leu Ala Met Ile Arg Thr Arg Ile Gln Ile Pro Ala Ala
        530                 535                 540

ccc atc cgg gaa gag gta gca gct gag atc ccc cat cgt tca ggc aag 1681
Pro Ile Arg Glu Glu Val Ala Ala Glu Ile Pro His Arg Ser Gly Lys
    545                 550                 555

gca gct ggt act gac ccc aac atg gct gac aat ggg gac ctg gac ctg 1729
Ala Ala Gly Thr Asp Pro Asn Met Ala Asp Asn Gly Asp Leu Asp Leu
560                 565                 570                 575

ggg ccg gta gag aca gag ccg ccc tat gcc tca cct aaa tac ctc cgt 1777
Gly Pro Val Glu Thr Glu Pro Pro Tyr Ala Ser Pro Lys Tyr Leu Arg
                580                 585                 590

aat ttc act ctg act gtc act gac tgt tac cgc agc tgg aac tcc gca 1825
Asn Phe Thr Leu Thr Val Thr Asp Cys Tyr Arg Ser Trp Asn Ser Ala
            595                 600                 605

ccc gga cct ttc cat ctt ttt cca cac aca ccc ttt gac cct gtg ctg 1873

Pro Gly Pro Phe His Leu Phe Pro His Thr Pro Phe Asp Pro Val Leu
        610                 615                 620

ccc tct gag gcc aaa ttc ctg ggc tca ggg act gga ttt cgg ccc atc 1921
Pro Ser Glu Ala Lys Phe Leu Gly Ser Gly Thr Gly Phe Arg Pro Ile
    625                 630                 635

ggt ggt ggg gct ggg ggc tct ggc aag gag ttc cag gca gcg ctt gga 1969
Gly Gly Gly Ala Gly Gly Ser Gly Lys Glu Phe Gln Ala Ala Leu Gly
640                 645                 650                 655

ggc aat gtc cag cgg gag cag ttc aca gtt gtg atg ctg acc tac gag 2017
Gly Asn Val Gln Arg Glu Gln Phe Thr Val Val Met Leu Thr Tyr Glu
                660                 665                 670

cgg gag gaa gtg ctc atg aac tcc ctg gag agg ctc aat ggc ctc ccc 2065
Arg Glu Glu Val Leu Met Asn Ser Leu Glu Arg Leu Asn Gly Leu Pro
            675                 680                 685

tac ctg aac aag gta gtg gtg gtg tgg aac tct ccc aag ctg ccc tcg 2113
Tyr Leu Asn Lys Val Val Val Val Trp Asn Ser Pro Lys Leu Pro Ser
        690                 695                 700

gag gac ctt ttg tgg cca gac att ggt gtc ccc atc atg gtt gtc cgt 2161
Glu Asp Leu Leu Trp Pro Asp Ile Gly Val Pro Ile Met Val Val Arg
    705                 710                 715

act gag aag aac agt ttg aac aat cgg ttc ttg ccc tgg aat gag ata     2209
Thr Glu Lys Asn Ser Leu Asn Asn Arg Phe Leu Pro Trp Asn Glu Ile
720                 725                 730                 735

gag aca gag gca ata ttg tcc atc gat gac gat gcc cac ctc cgc cat     2257
Glu Thr Glu Ala Ile Leu Ser Ile Asp Asp Asp Ala His Leu Arg His
                740                 745                 750

gat gaa atc atg ttc ggg ttt cgg gtg tgg aga gag gcg cgt gat cgc     2305
Asp Glu Ile Met Phe Gly Phe Arg Val Trp Arg Glu Ala Arg Asp Arg
            755                 760                 765

att gtg ggg ttc cct ggc cgg tac cat gcg tgg gac atc cct cac cag     2353
Ile Val Gly Phe Pro Gly Arg Tyr His Ala Trp Asp Ile Pro His Gln
        770                 775                 780

tcc tgg ctc tac aac tcc aac tac tcc tgt gag ctg tcc atg gtg ctg     2401
Ser Trp Leu Tyr Asn Ser Asn Tyr Ser Cys Glu Leu Ser Met Val Leu
    785                 790                 795

acg ggt gct gcc ttc ttt cac aag tat tac gcc tac ctg tat tct tat     2449
Thr Gly Ala Ala Phe Phe His Lys Tyr Tyr Ala Tyr Leu Tyr Ser Tyr
800                 805                 810                 815

gtg atg ccc cag gcc atc cga gac atg gtg gat gag tat atc aac tgt     2497

Val Met Pro Gln Ala Ile Arg Asp Met Val Asp Glu Tyr Ile Asn Cys
                820                 825                 830

gag gat atc gcc atg aac ttc ctt gtc tcc cac atc aca cgg aag ccc 2545
Glu Asp Ile Ala Met Asn Phe Leu Val Ser His Ile Thr Arg Lys Pro
            835                 840                 845

ccc atc aag gtg aca tcg agg tgg act ttt cga tgc ccg ggg tgc cct 2593
Pro Ile Lys Val Thr Ser Arg Trp Thr Phe Arg Cys Pro Gly Cys Pro
        850                 855                 860

cag gcc ctg tcc cac gat gac tct cac ttt cat gag cgg cac aag tgt 2641
Gln Ala Leu Ser His Asp Asp Ser His Phe His Glu Arg His Lys Cys
    865                 870                 875

atc aac ttt ttt gtc aag gtg tac ggc tat atg cct ctc ctg tac aca 2689
Ile Asn Phe Phe Val Lys Val Tyr Gly Tyr Met Pro Leu Leu Tyr Thr
880                 885                 890                 895

cag ttt agg gtg gac tct gtg ctc ttc aag acc cgc ctg ccc cat gac 2737
Gln Phe Arg Val Asp Ser Val Leu Phe Lys Thr Arg Leu Pro His Asp
                900                 905                 910

aag acc aag tgc ttc aag ttc atc tagggccttg ccagttctga ggagaagaca
2791
Lys Thr Lys Cys Phe Lys Phe Ile

            915

gtgagcagag tgaggggagt cacccccaag gttcccaagg tgttgaaggt ccttggggac
2851

atcgtgggca gggcccaggc cctttgcttg gagaagagca gggagagtag aaagggatgg
2911

ctgtctttat tttgaagtca gccgcactgg gcctggaatc ctggtcagca gactcagggc
2971

accgactaat ggcgaacact gaggactgtt catgagcccg ggacagctgg ttcccggttt
3031

ttaaattcag aacagcattt actatttaaa gagagagttt cacatctgcc atccaaggct
3091

tatttatatg tgcgtatatg tacacacata tgtgtatata catgtatatg cacgcacaca
3151

cacacacaca cacacacaca cacacacaca cacacacagc ggccgcg
3198

<210> 4
<211> 919

<212> PRT

<213> Rattus norvegicus

<400> 4

Met Thr Gly Tyr Thr Met Leu Arg Asn Gly Gly Val Gly Asn Gly Gly

1               5                   10                  15

Gln Thr Cys Met Leu Arg Trp Ser Asn Arg Ile Arg Leu Thr Trp Leu

            20                  25                  30

Ser Phe Thr Leu Phe Ile Ile Leu Val Phe Phe Pro Leu Ile Ala His

        35                  40                  45

Tyr Tyr Leu Thr Thr Leu Asp Glu Ala Asp Glu Ala Gly Lys Arg Ile

    50                  55                  60

Phe Gly Pro Arg Ala Gly Asn Glu Leu Cys Glu Val Lys His Val Leu

65                  70                  75                  80

Asp Leu Cys Arg Ile Arg Glu Ser Val Ser Glu Glu Leu Leu Gln Leu

                85                  90                  95

Glu Ala Lys Arg Gln Glu Leu Asn Ser Glu Ile Ala Lys Leu Asn Leu

            100                 105                 110

Lys Ile Glu Ala Cys Lys Lys Ser Ile Glu Asn Ala Lys Gln Asp Leu

        115                 120                 125

Leu Gln Leu Lys Asn Val Ile Ser Gln Thr Glu His Ser Tyr Lys Glu

    130                 135                 140

Leu Met Ala Gln Asn Gln Pro Lys Leu Ser Leu Pro Ile Arg Leu Leu

145                 150                 155                 160

Pro Glu Lys Asp Asp Ala Gly Leu Pro Pro Pro Lys Val Thr Arg Gly

                165                 170                 175

Cys Arg Leu His Asn Cys Phe Asp Tyr Ser Arg Cys Pro Leu Thr Ser

            180                 185                 190

Gly Phe Pro Val Phe Val Tyr Asp Ser Asp Gln Phe Ala Phe Gly Ser

        195                 200                 205

Tyr Leu Asp Pro Leu Val Lys Gln Ala Phe Gln Ala Thr Val Arg Ala

    210                 215                 220

Asn Val Tyr Val Thr Glu Asn Ala Ala Ile Ala Cys Leu Tyr Val Val

225                 230                 235                 240

Leu Val Gly Glu Ile Gln Glu Pro Ala Val Leu Gln Pro Ala Asp Leu

                245                 250                 255

Glu Lys Gln Leu His Ser Leu Pro His Trp Arg Thr Asp Gly His Asn
            260                 265                 270
His Val Ile Ile Asn Leu Ser Arg Lys Ser Asp Thr Gln Asn Leu Leu
        275                 280                 285
Tyr Asn Val Ser Thr Gly Arg Ala Met Val Ala Gln Ser Thr Phe Tyr
    290                 295                 300
Ala Ala Gln Tyr Arg Ala Gly Phe Asp Leu Val Val Ser Pro Leu Val
305                 310                 315                 320
His Ala Met Ser Glu Pro Asn Phe Met Glu Ile Pro Pro Gln Val Pro
                325                 330                 335
Val Lys Arg Lys Tyr Leu Phe Thr Phe Gln Gly Glu Lys Ile Glu Ser
            340                 345                 350
Leu Arg Ser Ser Leu Gln Glu Ala Arg Ser Phe Glu Glu Glu Met Glu
        355                 360                 365
Gly Asp Pro Pro Ala Asp Tyr Asp Asp Arg Ile Ile Ala Thr Leu Lys
    370                 375                 380
Ala Val Gln Asp Ser Lys Leu Asp Gln Val Leu Val Glu Phe Thr Cys
385                 390                 395                 400

Lys Asn Gln Pro Lys Pro Ser Leu Pro Thr Glu Trp Ala Leu Cys Gly
                405                 410                 415
Glu Arg Glu Asp Arg Leu Glu Leu Leu Lys Leu Ser Thr Phe Ala Leu
            420                 425                 430
Ile Ile Thr Pro Gly Asp Pro Ser Leu Leu Ile Ser Ser Gly Cys Ala
        435                 440                 445
Thr Arg Leu Phe Glu Ala Leu Glu Val Gly Ala Val Pro Val Val Leu
    450                 455                 460
Gly Glu Gln Val Gln Leu Pro Tyr His Asp Met Leu Gln Trp Asn Glu
465                 470                 475                 480
Ala Ala Leu Val Val Pro Lys Pro Arg Val Thr Glu Val His Phe Leu
                485                 490                 495
Leu Arg Ser Leu Ser Asp Ser Asp Leu Leu Ala Met Arg Arg Gln Gly
            500                 505                 510
Arg Phe Leu Trp Glu Thr Tyr Phe Ser Thr Ala Asp Ser Ile Phe Asn
        515                 520                 525
Thr Val Leu Ala Met Ile Arg Thr Arg Ile Gln Ile Pro Ala Ala Pro

530                 535                 540

Ile Arg Glu Glu Val Ala Ala Glu Ile Pro His Arg Ser Gly Lys Ala
545                 550                 555                 560

Ala Gly Thr Asp Pro Asn Met Ala Asp Asn Gly Asp Leu Asp Leu Gly
                565                 570                 575

Pro Val Glu Thr Glu Pro Pro Tyr Ala Ser Pro Lys Tyr Leu Arg Asn
            580                 585                 590

Phe Thr Leu Thr Val Thr Asp Cys Tyr Arg Ser Trp Asn Ser Ala Pro
        595                 600                 605

Gly Pro Phe His Leu Phe Pro His Thr Pro Phe Asp Pro Val Leu Pro
    610                 615                 620

Ser Glu Ala Lys Phe Leu Gly Ser Gly Thr Gly Phe Arg Pro Ile Gly
625                 630                 635                 640

Gly Gly Ala Gly Gly Ser Gly Lys Glu Phe Gln Ala Ala Leu Gly Gly
                645                 650                 655

Asn Val Gln Arg Glu Gln Phe Thr Val Val Met Leu Thr Tyr Glu Arg
            660                 665                 670

Glu Glu Val Leu Met Asn Ser Leu Glu Arg Leu Asn Gly Leu Pro Tyr
        675                 680                 685
Leu Asn Lys Val Val Val Val Trp Asn Ser Pro Lys Leu Pro Ser Glu
    690                 695                 700
Asp Leu Leu Trp Pro Asp Ile Gly Val Pro Ile Met Val Val Arg Thr
705                 710                 715                 720
Glu Lys Asn Ser Leu Asn Asn Arg Phe Leu Pro Trp Asn Glu Ile Glu
                725                 730                 735
Thr Glu Ala Ile Leu Ser Ile Asp Asp Asp Ala His Leu Arg His Asp
            740                 745                 750
Glu Ile Met Phe Gly Phe Arg Val Trp Arg Glu Ala Arg Asp Arg Ile
        755                 760                 765
Val Gly Phe Pro Gly Arg Tyr His Ala Trp Asp Ile Pro His Gln Ser
    770                 775                 780
Trp Leu Tyr Asn Ser Asn Tyr Ser Cys Glu Leu Ser Met Val Leu Thr
785                 790                 795                 800
Gly Ala Ala Phe Phe His Lys Tyr Tyr Ala Tyr Leu Tyr Ser Tyr Val
                805                 810                 815

Met Pro Gln Ala Ile Arg Asp Met Val Asp Glu Tyr Ile Asn Cys Glu
            820                 825                 830
Asp Ile Ala Met Asn Phe Leu Val Ser His Ile Thr Arg Lys Pro Pro
        835                 840                 845
Ile Lys Val Thr Ser Arg Trp Thr Phe Arg Cys Pro Gly Cys Pro Gln
    850                 855                 860
Ala Leu Ser His Asp Asp Ser His Phe His Glu Arg His Lys Cys Ile
865                 870                 875                 880
Asn Phe Phe Val Lys Val Tyr Gly Tyr Met Pro Leu Leu Tyr Thr Gln
                885                 890                 895
Phe Arg Val Asp Ser Val Leu Phe Lys Thr Arg Leu Pro His Asp Lys
            900                 905                 910
Thr Lys Cys Phe Lys Phe Ile
        915
<210> 5
<211> 919
<212> PRT

<213> Homo sapiens

<400> 5
<210> 6
<211> 718
<212> PRT
<213> Homo sapiens

<400> 6

<210> 7
<211> 746
<212> PRT
<213> Homo sapiens

<400> 7
Met Gln Ala Lys Lys Arg Tyr Phe Ile Leu Leu Ser Ala Gly Ser Cys
1               5                   10                  15

Leu Ala Leu Leu Phe Tyr Phe Gly Gly Leu Gln Phe Arg Ala Ser Arg
            20                  25                  30

Ser His Ser Arg Arg Glu Glu His Ser Gly Arg Asn Gly Leu His His
        35                  40                  45

Pro Ser Pro Asp His Phe Trp Pro Arg Phe Pro Glu Pro Leu Arg Pro
    50                  55                  60

Phe Val Pro Trp Asp Gln Leu Glu Asn Glu Asp Ser Ser Val His Ile
65                  70                  75                  80

Ser Pro Arg Gln Lys Arg Asp Ala Asn Ser Ser Ile Tyr Lys Gly Lys
                85                  90                  95

Lys Cys Arg Met Glu Ser Cys Phe Asp Phe Thr Leu Cys Lys Lys Asn
            100                 105                 110

Gly Phe Lys Val Tyr Val Tyr Pro Gln Gln Lys Gly Glu Lys Ile Ala
        115                 120                 125

Glu Ser Tyr Gln Asn Ile Leu Ala Ala Ile Glu Gly Ser Arg Phe Tyr
    130                 135                 140

Thr Ser Asp Pro Ser Gln Ala Cys Leu Phe Val Leu Ser Leu Asp Thr
145                 150                 155                 160

Leu Asp Arg Asp Gln Leu Ser Pro Gln Tyr Val His Asn Leu Arg Ser
                165                 170                 175

Lys Val Gln Ser Leu His Leu Trp Asn Asn Gly Arg Asn His Leu Ile
            180                 185                 190

Phe Asn Leu Tyr Ser Gly Thr Trp Pro Asp Tyr Thr Glu Asp Val Gly
        195                 200                 205

Phe Asp Ile Gly Gln Ala Met Leu Ala Lys Ala Ser Ile Ser Thr Glu
    210                 215                 220
Asn Phe Arg Pro Asn Phe Asp Val Ser Ile Pro Leu Phe Ser Lys Asp
225                 230                 235                 240
His Pro Arg Thr Gly Gly Glu Arg Gly Phe Leu Lys Phe Asn Thr Ile
                245                 250                 255
Pro Pro Leu Arg Lys Tyr Met Leu Val Phe Lys Gly Lys Arg Tyr Leu
            260                 265                 270
Thr Gly Ile Gly Ser Asp Thr Arg Asn Ala Leu Tyr His Val His Asn
        275                 280                 285
Gly Glu Asp Val Val Leu Leu Thr Thr Cys Lys His Gly Lys Asp Trp
    290                 295                 300
Gln Lys His Lys Asp Ser Arg Cys Asp Arg Asp Asn Thr Glu Tyr Glu
305                 310                 315                 320
Lys Tyr Asp Tyr Arg Glu Met Leu His Asn Ala Thr Phe Cys Leu Val
                325                 330                 335
Pro Arg Gly Arg Arg Leu Gly Ser Phe Arg Phe Leu Glu Ala Leu Gln

            340                 345                 350

Ala Ala Cys Val Pro Val Met Leu Ser Asn Gly Trp Glu Leu Pro Phe
        355                 360                 365

Ser Glu Val Ile Asn Trp Asn Gln Ala Ala Val Ile Gly Asp Glu Arg
    370                 375                 380

Leu Leu Leu Gln Ile Pro Ser Thr Ile Arg Ser Ile His Gln Asp Lys
385                 390                 395                 400

Ile Leu Ala Leu Arg Gln Gln Thr Gln Phe Leu Trp Glu Ala Tyr Phe
                405                 410                 415

Ser Ser Val Glu Lys Ile Val Leu Thr Thr Leu Glu Ile Ile Gln Asp
            420                 425                 430

Arg Ile Phe Lys His Ile Ser Arg Asn Ser Leu Ile Trp Asn Lys His
        435                 440                 445

Pro Gly Gly Leu Phe Val Leu Pro Gln Tyr Ser Ser Tyr Leu Gly Asp
    450                 455                 460

Phe Pro Tyr Tyr Tyr Ala Asn Leu Gly Leu Lys Pro Pro Ser Lys Phe
465                 470                 475                 480

Thr Ala Val Ile His Ala Val Thr Pro Leu Val Ser Gln Ser Gln Pro
                485                 490                 495
Val Leu Lys Leu Leu Val Ala Ala Ala Lys Ser Gln Tyr Cys Ala Gln
            500                 505                 510
Ile Ile Val Leu Trp Asn Cys Asp Lys Pro Leu Pro Ala Lys His Arg
        515                 520                 525
Trp Pro Ala Thr Ala Val Pro Val Val Val Ile Glu Gly Glu Ser Lys
    530                 535                 540
Val Met Ser Ser Arg Phe Leu Pro Tyr Asp Asn Ile Ile Thr Asp Ala
545                 550                 555                 560
Val Leu Ser Leu Asp Glu Asp Thr Val Leu Ser Thr Thr Glu Val Asp
                565                 570                 575
Phe Ala Phe Thr Val Trp Gln Ser Phe Pro Glu Arg Ile Val Gly Tyr
            580                 585                 590
Pro Ala Arg Ser His Phe Trp Asp Asn Ser Lys Glu Arg Trp Gly Tyr
        595                 600                 605
Thr Ser Lys Trp Thr Asn Asp Tyr Ser Met Val Leu Thr Gly Ala Ala
    610                 615                 620

Ile Tyr His Lys Tyr Tyr His Tyr Leu Tyr Ser His Tyr Leu Pro Ala
625                 630                 635                 640
Ser Leu Lys Asn Met Val Asp Gln Leu Ala Asn Cys Glu Asp Ile Leu
                645                 650                 655
Met Asn Phe Leu Val Ser Ala Val Thr Lys Leu Pro Pro Ile Lys Val
            660                 665                 670
Thr Gln Lys Lys Gln Tyr Lys Glu Thr Met Met Gly Gln Thr Ser Arg
        675                 680                 685
Ala Ser Arg Trp Ala Asp Pro Asp His Phe Ala Gln Arg Gln Ser Cys
    690                 695                 700
Met Asn Thr Phe Ala Ser Trp Phe Gly Tyr Met Pro Leu Ile His Ser
705                 710                 715                 720
Gln Met Arg Leu Asp Pro Val Leu Phe Lys Asp Gln Val Ser Ile Leu
                725                 730                 735
Arg Lys Lys Tyr Arg Asp Ile Glu Arg Leu
            740                 745

<210> 8

<211> 676

<212> PRT

<213> Homo sapiens

<400> 8

Met Gln Ser Trp Arg Arg Arg Lys Ser Leu Trp Leu Ala Leu Ser Ala
1               5                   10                  15

Ser Trp Leu Leu Leu Val Leu Leu Gly Gly Phe Ser Leu Leu Arg Leu
            20                  25                  30

Ala Leu Pro Pro Arg Pro Arg Pro Gly Ala Ser Gln Gly Trp Pro Arg
        35                  40                  45

Trp Leu Asp Ala Glu Leu Leu Gln Ser Phe Ser Gln Pro Gly Glu Leu
    50                  55                  60

Pro Glu Asp Ala Val Ser Pro Pro Gln Ala Pro His Gly Gly Ser Cys
65                  70                  75                  80

Asn Trp Glu Ser Cys Phe Asp Thr Ser Lys Cys Arg Gly Asp Gly Leu
                85                  90                  95

Lys Val Phe Val Tyr Pro Ala Val Gly Thr Ile Ser Glu Thr His Arg
            100                 105                 110

Arg Ile Leu Ala Ser Ile Glu Gly Ser Arg Phe Tyr Thr Phe Ser Pro
        115                 120                 125
Ala Gly Ala Cys Leu Leu Leu Leu Leu Ser Leu Asp Ala Gln Thr Gly
    130                 135                 140
Glu Cys Ser Ser Met Pro Leu Gln Trp Asn Arg Gly Arg Asn His Leu
145                 150                 155                 160
Val Leu Arg Leu His Pro Ala Pro Cys Pro Arg Thr Phe Gln Leu Gly
                165                 170                 175
Gln Ala Met Val Ala Glu Ala Ser Pro Thr Val Asp Ser Phe Arg Pro
            180                 185                 190
Gly Phe Asp Val Ala Leu Pro Phe Leu Pro Glu Ala His Pro Leu Arg
        195                 200                 205
Gly Gly Ala Pro Gly Gln Leu Arg Gln His Ser Pro Gln Pro Gly Val
    210                 215                 220
Ala Leu Leu Ala Leu Glu Glu Glu Arg Gly Gly Trp Arg Thr Ala Asp
225                 230                 235                 240
Thr Gly Ser Ser Ala Cys Pro Trp Asp Gly Arg Cys Glu Gln Asp Pro

                245                 250                 255

Gly Pro Gly Gln Thr Gln Arg Gln Glu Thr Leu Pro Asn Ala Thr Phe

            260                 265                 270

Cys Leu Ile Ser Gly His Arg Pro Glu Ala Ala Ser Arg Phe Leu Gln

        275                 280                 285

Ala Leu Gln Ala Gly Cys Ile Pro Val Leu Leu Ser Pro Arg Trp Glu

    290                 295                 300

Leu Pro Phe Ser Glu Val Ile Asp Trp Thr Lys Ala Ala Ile Val Ala

305                 310                 315                 320

Asp Glu Arg Leu Pro Leu Gln Val Leu Ala Ala Leu Gln Glu Met Ser

                325                 330                 335

Pro Ala Arg Val Leu Ala Leu Arg Gln Gln Thr Gln Phe Leu Trp Asp

            340                 345                 350

Ala Tyr Phe Ser Ser Val Glu Lys Val Ile His Thr Thr Leu Glu Val

        355                 360                 365

Ile Gln Asp Arg Ile Phe Gly Thr Ser Ala Asn Pro Ser Leu Leu Trp

    370                 375                 380

Asn Ser Pro Pro Gly Ala Leu Leu Ala Leu Ser Thr Phe Ser Thr Ser
385                 390                 395                 400
Pro Gln Asp Phe Pro Phe Tyr Tyr Leu Gln Gln Gly Ser Arg Pro Glu
                405                 410                 415
Gly Arg Phe Ser Ala Leu Ile Trp Val Gly Pro Pro Gly Gln Pro Pro
            420                 425                 430
Leu Lys Leu Ile Gln Ala Val Ala Gly Ser Gln His Cys Ala Gln Ile
        435                 440                 445
Leu Val Leu Trp Ser Asn Glu Arg Pro Leu Pro Ser Arg Trp Pro Glu
    450                 455                 460
Thr Ala Val Pro Leu Thr Val Ile Asp Gly His Arg Lys Val Ser Asp
465                 470                 475                 480
Arg Phe Tyr Pro Tyr Ser Thr Ile Arg Thr Asp Ala Ile Leu Ser Leu
                485                 490                 495
Asp Ala Arg Ser Ser Leu Ser Thr Ser Glu Val Asp Phe Ala Phe Leu
            500                 505                 510
Val Trp Gln Ser Phe Pro Glu Arg Met Val Gly Phe Leu Thr Ser Ser
        515                 520                 525

His Phe Trp Asp Glu Ala His Gly Gly Trp Gly Tyr Thr Ala Glu Arg
    530                 535                 540

Thr Asn Glu Phe Ser Met Val Leu Thr Thr Ala Ala Phe Tyr His Arg
545                 550                 555                 560

Tyr Tyr His Thr Leu Phe Thr His Ser Leu Pro Lys Ala Leu Arg Thr
                565                 570                 575

Leu Ala Asp Glu Ala Pro Thr Cys Val Asp Val Leu Met Asn Phe Ile
            580                 585                 590

Val Ala Ala Val Thr Lys Leu Pro Pro Ile Lys Val Pro Tyr Gly Lys
        595                 600                 605

Gln Arg Gln Glu Ala Ala Pro Leu Ala Pro Gly Gly Pro Gly Pro Arg
    610                 615                 620

Pro Lys Pro Pro Ala Pro Ala Pro Asp Cys Ile Asn Gln Ile Ala Ala
625                 630                 635                 640

Ala Phe Gly His Met Pro Leu Leu Ser Ser Arg Leu Arg Leu Asp Pro
                645                 650                 655

Val Leu Phe Lys Asp Pro Val Ser Val Gln Arg Lys Lys Tyr Arg Ser

            660                 665                 670

Leu Glu Lys Pro

        675

<210> 9

<211> 330

<212> PRT

<213> Homo sapiens

<400> 9

Met Arg Cys Cys His Ile Cys Lys Leu Pro Gly Arg Val Met Gly Ile

1               5                   10                  15

Arg Val Leu Arg Leu Ser Leu Val Val Ile Leu Val Leu Leu Leu Val

            20                  25                  30

Ala Gly Ala Leu Thr Ala Leu Leu Pro Ser Val Lys Glu Asp Lys Met

        35                  40                  45

Leu Met Leu Arg Arg Glu Ile Lys Ser Gln Gly Lys Ser Thr Met Asp

    50                  55                  60

Ser Phe Thr Leu Ile Met Gln Thr Tyr Asn Arg Thr Asp Leu Leu Leu

65                  70                  75                  80

Lys Leu Leu Asn His Tyr Gln Ala Val Pro Asn Leu His Lys Val Ile
                85                  90                  95

Val Val Trp Asn Asn Ile Gly Glu Lys Ala Pro Asp Glu Leu Trp Asn
            100                 105                 110

Ser Leu Gly Pro His Pro Ile Pro Val Ile Phe Lys Gln Gln Thr Ala
        115                 120                 125

Asn Arg Met Arg Asn Arg Leu Gln Val Phe Pro Glu Leu Glu Thr Asn
    130                 135                 140

Ala Val Leu Met Val Asp Asp Asp Thr Leu Ile Ser Thr Pro Asp Leu
145                 150                 155                 160

Val Phe Ala Phe Ser Val Trp Gln Gln Phe Pro Asp Gln Ile Val Gly
                165                 170                 175

Phe Val Pro Arg Lys His Val Ser Thr Ser Ser Gly Ile Tyr Ser Tyr
            180                 185                 190

Gly Ser Phe Glu Met Gln Ala Pro Gly Ser Gly Asn Gly Asp Gln Tyr
        195                 200                 205

Ser Met Val Leu Ile Gly Ala Ser Phe Phe Asn Ser Lys Tyr Leu Glu

    210                 215                 220

Leu Phe Gln Arg Gln Pro Ala Ala Val His Ala Leu Ile Asp Asp Thr
225                 230                 235                 240

Gln Asn Cys Asp Asp Ile Ala Met Asn Phe Ile Ile Ala Lys His Ile
                245                 250                 255

Gly Lys Thr Ser Gly Ile Phe Val Lys Pro Val Asn Met Asp Asn Leu
            260                 265                 270

Glu Lys Glu Thr Asn Ser Gly Tyr Ser Gly Met Trp His Arg Ala Glu
        275                 280                 285

His Ala Leu Gln Arg Ser Tyr Cys Ile Asn Lys Leu Val Asn Ile Tyr
    290                 295                 300

Asp Ser Met Pro Leu Arg Tyr Ser Asn Ile Met Ile Ser Gln Phe Gly
305                 310                 315                 320

Phe Pro Tyr Ala Asn Tyr Lys Arg Lys Ile
                325                 330

## Description

### Technical field

The present invention relates to a novel protein that binds to the Reg protein, gene thereof, and production and uses of this protein and gene.

### Background art

β cells of pancreatic Langerhans' islet produce insulin, the sole blood hypoglycemic factor in the living body. So far, it was thought that once pancreatic β cell numbers are decreased following some damage, these cells would not easily regenerate and grow. This is considered to be an important factor in the onset of diabetes, and also the reason why a cause-based fundamental diabetes therapy cannot be established.

Conventionally, in the treatment of diabetes, insulin or an oral anti-diabetic drug of the sulfonylurea-type is administrated. However, insulin administration is a symptomatic therapy, and it is also difficult to maintain the physiological concentration of blood insulin. Furthermore, when considering the treatment of diabetic complications such as arteriosclerosis, neuropathy, and the progression of retinopathy, this therapy had its limitations. Moreover, prolonged use of oral anti-diabetic drugs caused side effects such as coronary arteriosclerosis, or decrease in insulin-secreting ability thought to be caused by an excessive load to the pancreas.

The present inventors have previously demonstrated the mechanism of pancreatic β-cell damage and its prevention (H. Yamamoto, et al., Nature 294, 284(1981); Y. Uchigata, et al., J. Biol. Chem. 257, 6084(1982); Y. Uchigata, et al., Diabetes 32, 316(1983); H. Okamoto, Bioassays 2, 15 (1985) ; H. Okamoto, J. Mol. Med. 77, 74(1999)). Further, the present inventors have succeeded in the regeneration and growth of pancreatic β cells (T. Watanabe et al., Proc. Natl. Acad. Sci. USA 91, 3589 (1994); Yonemura, Y. et al. (1984) Diabetes 33, 401-404), and isolated a gene expressing specifically during the regeneration, named Reg (Regenerating gene) (H. Okamoto, J. Mol. Med. 77, 74 (1999); K. Terazono, et al., J. Biol. Chem. 263, 2111 (1988); K. Terazono, T. Watanabe, Y. Yonemura, in Molecular biology of the islets of Langerhans', H. Okamoto, Ed. (Cambridge University Press, Cambridge, 1990), pp. 301-313; K. Terazono et al., Diabetologia 33, 250 (1990); T. Watanabe et al., Proc. Natl. Acad. Sci. USA 91, 3589 (1994)). Moreover, the present inventors elucidated that Reg protein, the gene-product of Reg gene, is a regeneration growth factor of pancreatic β cells, and showed the possibility of treating diabetes by the administration of the Reg protein, the activation of Reg gene, or the introduction of Reg gene, by using a diabetes model animal (Watanabe, T. et al. (1994) Proc. Natl. Acad. Sci. USA 91, 3589-3592; Gross, D.J. et al. (1998) Endocrinology 139, 2369-2374; Okamoto, H. (1999) J. Mol. Med. 77, 74-79). From these analysis, administration of Reg protein was found to induce the regeneration and growth of β-cells, thereby increasing β-cell mass and amelioration of diabetes in 90% of pancreatectomized rats and in non-obese diabetic mice. However, it was unknown as to which proteins interact with the Reg protein to exert its functions.

Reg protein is expected to be applied to diabetes treatment as a growth factor of pancreatic β cells, to make up for the weak-points of insulin administration. However, a lot of technical issues still exist when it comes to clinical application, such as that oral administration of Reg protein is difficult due to its high-molecular weight, and furthermore, the in vivo targeting of a high-molecular weight protein is difficult.

### Disclosure of the Invention

An objective of the present invention is to provide a novel protein binding to Reg protein, gene thereof, and methods of production and uses of the protein and gene. Especially, the protein of the present invention is useful for the development of a novel therapeutic drug for diabetes.

In order to analyze the function of Reg protein towards pancreatic β cell-lineage cells, the present inventors conducted an experiment in which a recombinant Reg protein produced in yeast was added to the rat insulinoma cell-derived cell line, RINm5F. As a result, it was revealed that the addition of Reg protein increases incorporation of 5'-bromo-2'-deoxyuridine (BrdU) in RINm5F cells significantly, and that the growth of these cells is promoted by Reg protein. Next, the present inventors labeled the Reg protein with ¹²⁵I and added it to RINm5F cells to analyze the binding activity. As a result, concentration-dependent binding of Reg protein to RINm5F cells was observed, and the binding was thought to be specific since it was inhibited by an excess amount of unlabeled Reg protein. These results suggest that pancreatic β cells express a Reg protein receptor and the binding of this receptor to Reg protein promotes cell growth.

To isolate a Reg-binding protein that functions as a Reg protein receptor, the present inventors constructed an expression cDNA library from rat pancreatic Langerhans' islet polyA (+) RNA by a phage vector and screened genes encoding a Reg-binding protein by West-Western blotting method using a labeled Reg protein. As a result, a novel cDNA encoding a protein comprising 364 amino acids was successfully isolated. This cDNA was inserted into a mammalian cell-expression vector, and expressed in COS-7 cells. Addition of recombinant Reg protein to these cells confirmed that Reg protein bound specifically to COS-7 cells.

Using this cDNA as a probe, the present inventors succeeded in isolating another cDNA encoding a Reg-binding protein by screening a rat pancreas Langerhans' islet cDNA library. The cDNA was encoding a cell surface protein comprising 919 amino acids. When the cDNA was expressed in mammalian cells, the protein was expressed on the cell surface and the cells bound to Reg protein with a high affinity. The addition of Reg protein induced the incorporation of BrdU in RINmSF β cells transfected with the cDNA, and the cell number was increased. From these results, it was shown that the Reg-binding protein encoded by the isolated cDNA was a receptor for Reg protein, and mediated cell proliferation signals in pancreatic β cells. Moreover, it was revealed that apoptosis is induced in RINm5F cells highly expressing the Reg-binding protein by the addition of a high concentration of Reg protein.

From these facts, it can be envisaged that the Reg-binding protein transduces signals of Reg protein, and by regulating cell proliferation, and such, of pancreatic β cells, the Reg-binding protein regulates pancreatic β cell mass. The Reg-binding protein of the present invention and gene thereof would be useful tools for elucidating the etiological mechanism of diabetes, and these can also be applied to the development of anti-diabetic drugs.

The present invention relates to a Reg protein-binding protein, gene thereof, and methods for producing the protein and gene, and uses thereof, more specifically to:
(1) a DNA according to any one of (a) to (i),
   (a) a DNA encoding a protein comprising the amino acid sequence of SEQ ID NO: 2,
   (b) a DNA comprising the coding sequence of the nucleotide sequence of SEQ ID NO: 1,
   (c) a DNA encoding a protein comprising an amino acid sequence in which one or more amino acids of the amino acid sequence of SEQ ID NO: 2 have been substituted, deleted, inserted and/or added, wherein said DNA encodes a protein having the activity of binding to Reg protein,
   (d) a DNA hybridizing to a DNA comprising the nucleotide sequence of SEQ ID NO: 1, wherein said DNA encodes a protein having the activity of binding to Reg protein,
   (e) a DNA encoding a protein comprising the amino acid sequence of SEQ ID NO: 4,
   (f) a DNA comprising the coding region of the nucleotide sequence of SEQ ID NO: 3,
   (g) a DNA encoding a protein comprising the amino acid sequence in which one or more amino acids of the amino acid sequence of SEQ ID NO: 4 have been substituted, deleted, inserted and/or added, wherein the DNA encodes a protein having the activity of binding to Reg protein,
   (h) a DNA hybridizing to a DNA comprising the nucleotide sequence of SEQ ID NO: 3, wherein said DNA encodes a protein having the activity of binding to Reg protein,
   (i) a DNA encoding a partial peptide of a protein comprising the amino acid sequence of SEQ ID NO: 2 or SEQ ID NO: 4;
(2) a protein or peptide encoded by the DNA according to (1);
(3) a vector into which the DNA according to (1) has been inserted;
(4) a host cell carrying the vector according to (3);
(5) a method for producing the protein or peptide according to (2), wherein said method comprises the following steps of,
   (a) culturing the cell according to (4), and,
   (b) recovering the recombinant protein expressed by the cell from the cultured cell or from the culture supernatant;
(6) an antibody against the protein or peptide according to (2);
(7) a polynucleotide comprising at least 15 nucleotides, wherein said polynucleotide hybridizes with a DNA selected from the group consisting of SEQ ID NO: 1, SEQ ID NO: 3, and DNA complementary thereto;
(8) a method of screening for a compound that binds to the protein or peptide according to (2), wherein said method comprises the following steps of,
   (a) contacting the protein or peptide with a test sample,
   (b) detecting the binding of the test sample to the protein or peptide, and,
   (c) selecting a compound that binds to the protein or peptide;
(9) a method of screening for a compound that inhibits the binding of Reg protein to the protein or peptide according to (2), wherein said method comprises the following steps of,
   (a) contacting Reg protein with the protein or peptide according to (2) in the presence of a test sample,
   (b) detecting the binding of Reg protein to the protein or peptide according to (2), and,
   (c) selecting a compound that decreases the binding;
(10) a compound isolated by the method according to (9), wherein said compound inhibits the binding of Reg protein to the protein or peptide according to (2);
(11) a method of screening for a compound that promotes or inhibits signal transduction caused by an activation of the protein according to (2), wherein said method comprises the following steps of,
   (a) contacting Reg protein with a cell expressing the protein according to (2) on the cell surface, in the presence of a test sample,
   (b) detecting a change of the cell in response to the stimulation by Reg protein,
   (c) selecting a compound that enhances or suppresses the change of the cell as compared to when detected in the absence of the test sample;
(12) the method according to (11), wherein said change of the cell detected comprises a change in cell-proliferating activity or DNA-synthesizing activity of the cell;
(13) a compound isolated by the method according to (11) or (12), wherein said compound promotes or inhibits signal transduction caused by an activation of the protein according to (2);
(14) a pharmaceutical agent comprising the DNA according to (1), the protein or peptide according to (2), the vector according to (3), the antibody according to (6), or the compound according to (10) or (13);
(15) the pharmaceutical agent according to (14), wherein said pharmaceutical agent is selected from the group consisting of a Reg-binding agent, a regulator of intracellular signal transduction of cells responding to Reg protein, a cell growth regulator, a DNA synthesis regulator, and an apoptosis regulator; and,
(16) the pharmaceutical agent according to (14) or (15), wherein said pharmaceutical agent is an anti-diabetic drug.

The present invention relates to a novel protein expressed in the pancreas that binds to the Reg protein (Reg-binding protein). The nucleotide sequences of cDNAs of isolated rat "Reg-binding protein" and amino acid sequences encoded by these cDNA are described in SEQ ID NO: 1 and SEQ ID NO: 3, and SEQ ID NO: 2 and SEQ ID NO: 4, respectively.

One of the cDNA encoding the rat Reg-binding protein of the present invention (SEQ ID NO: 2) comprises an open reading frame encoding a protein comprising 364 amino acid residues (SEQ ID NO: 1). By a screening using this cDNA as probe, a cDNA encoding Reg-binding protein comprising an open reading frame (SEQ ID NO: 3) encoding a protein comprising 919 amino acid residues (SEQ ID NO: 4) could be isolated. The rat "Reg-binding protein" of the present invention is expressed on the cell surface and has a Reg protein-binding activity. As described above, the Reg protein is a regeneration growth factor that is specifically expressed when pancreatic β cells are regenerated, and the possibility of applying this protein and the gene thereof in treating diabetes has been suggested. It is thought that the Reg-binding protein of the present invention relates to the regulation of physiological functions of cells including growth regulation of pancreatic β cells, by functioning as a receptor of the Reg protein. Therefore, the Reg-binding protein of the present invention maybe useful as a research target for elucidating the mechanism that causes diabetes, or as a tool for developing a therapeutic agent against diseases involving pancreatic β cell functions (such as diabetes).

Recently, several Reg and Reg-related genes have been isolated, and these have been revealed to constitute a multigene family, the Reg family (H. Okamoto, J. Mol. Med. 77, 74 (1999); H. Okamoto, J. Hepatobiliary Pancreat. Surg. 6, 254 (1999); M. Unno et al., J. Biol. Chem. 268, 15974 (1993); Y. Narushima et al., Gene 185, 159 (1997); M. Abe et al., Gene 246, 111 (2000)). All the members of the Reg family show the conserved gene organization of 6 exons and 5 introns and 40-85% amino acid sequence homologies among the family with the conserved 6 cysteine residues forming 3 pairs of intramolecular S-S bonds (H. Okamoto, J. Mol. Med. 77, 74 (1999); H. Okamoto, J. Hepatobiliary Pancreat. Surg. 6, 254 (1999) ; M. Unno et al., J. Biol. Chem. 268, 15974 (1993); Y. Narushima et al., Gene 185, 159 (1997); T. Itoh et al., FEBS Lett. 272, 85 (1990); M. Abe et al., Gene 246, 111 (2000)). Based on the primary structures of Reg proteins, the members of the family are grouped into three subclasses, type I, II and III (H. Okamoto, J. Mol. Med. 77, 74 (1999); H. Okamoto, J. Hepatobiliary Pancreat. Surg. 6, 254 (1999); M. Unno et al., J. Biol. Chem. 268, 15974 (1993); Y. Narushima et al., Gene 185, 159 (1997); T. Watanabe et al., J. Biol. Chem. 265, 7432(1990); M. Abe et al., Gene 246, 111 (2000)). Type I Reg proteins, which include the rat and human Reg proteins used in the examples of the present invention, are expressed in regenerating pancreatic islets (H. Okamoto, J. Mol. Med. 77, 74 (1999); K. Terazono, et al., J. Biol. Chem. 263, 2111 (1988); K. Terazono, T. Watanabe, Y. Yonemura, in Molecular biology of the islets of Langerhans', H. Okamoto, Ed. (Cambridge University Press, Cambridge, 1990), pp. 301-313; K. Terazono et al., Diabetologia 33, 250 (1990); H. Okamoto, J. Hepatobiliary Pancreat. Surg. 6, 254 (1999)). Recently, type I Reg expression under pathological conditions has been reported in human colon cancer (T. Watanabe et al., J. Biol. Chem. 265, 7432 (1990); *M.* E. Zenilman et al., J. Gastrointest. Surg. 1, 194 (1997); F. R. Bernard-Perrone et al., J. Histochem. Cytochem. 47, 863 (1999)), and in rat gastric mucosa (H. Fukui et al., Gastroenterology 115, 1483 (1998)) and enterochromaffin-like cells (M. Asahara et al., Gastroenterology 111, 45 (1996)), and type III Reg proteins have also been suggested to be involved in cellular proliferation in intestinal Paneth cells (L. Christa et al., Am. J. Physiol. 271, G993 (1996)), hepatocellular carcinomas (L. Christa et al., Am. J. Physiol. 271, G993 (1996)), pancreatic acinar cells (L. Christa et al., Am. J. Physiol. 271, G993 (1996); E. M. Ortiz et al., Gastroenterology 114, 808 (1998)) and Schwann cells (J. F. Livesey et al., Nature 390, 614 (1997)). Therefore, the identified Reg receptor may function in various tissues and cells in physiological and pathological conditions as a receptor for the Reg family gene products.

As shown by findings described above, the protein of the present invention is useful for the development of a therapeutic agent for the treatment and prevention of not only diabetes, but also diseases such as gastrointestinal tumors (Asahara, M. et al., Gastroenterology 111, 45-55 (1996); Fukui, H. et al., Gastroenterology 115, 1483-1493 (1998)), neurodegeneration diseases (Livesy, F.J. et al., Nature 390, 614-618 (1997)), and pancreatitis (Christa, L. et al., Am. J. Phsiol. 271, G993-G1002 (1996); Ortiz, E. et al., Gastroenterology 114, 808-816 (1998)). Moreover, it is thought that Reg protein itself can be applied for the treatment when Reg protein-Reg-binding protein disorders, for example, overstimulation, occur in tumors and such, since the administration of the soluble form of Reg-binding protein can inhibit the overstimulation to suppress tumor growth, etc.

The present invention includes proteins structurally similar to rat "Reg-binding protein", as long as they have a binding activity to Reg protein. Structurally similar proteins include mutants of "Reg-binding protein" and "Reg-binding proteins" derived from other organisms.

One skilled in the art could readily prepare these proteins using, for example, well-known mutagenesis methods. Known methods for altering amino acids in proteins include Kunkel's method (Kunkel, T. A. (1985) Proc. Natl. Acad. Sci. USA 82, 488), Oligonucleotide -directed Dual Amber (ODA) method (Hashimoto-Gotoh, T. et al. (1995) Gene 152, 271-275), PCR-restriction enzyme method (Ito, W. et al. (1991) Gene 102, 67-70), ODA-PCR method (Hashimoto-Gotoh, T. et al. (1995) Gene 152, 271-275; Ito, W. et al. (1991) Gene 102, 67-70), etc. There is no restriction on the number of amino acid residues altered, but when artificially doing so, the number of amino acid residues altered is usually 50 or less, preferably 10 or less, and more preferably 5 or less.

Mutation of amino acids in proteins could occur spontaneously. Such proteins having amino acid sequences different from that of the natural rat "Reg-binding protein" due to artificial or spontaneous substitution, deletion, addition and/or insertion of amino acids, are also included in this invention as long as they have a binding activity to Reg protein.

An amino acid having properties similar to those of the substituted amino acid is preferably used for the substitution. For example, since Ala, Val, Leu, Ile, Pro, Met, Phe and Trp are, classified as non-polar amino acids, they are considered to have similar properties. Moreover, non-charged amino acids include Gly, Ser, Thr, Cys, Tyr, Asn, and Gln. Furthermore, acidic amino acids include Asp and Glu, while basic amino acids include Lys, Arg and His.

In the present invention, a protein that is deficient in amino acids of rat "Reg-binding protein" includes a protein comprising only the extracellular domain. Moreover, a protein comprising an amino acid addition to rat "Reg-binding protein" includes a fusion protein of rat "Reg-binding protein" and another peptide.

Proteins structurally similar to the rat "Reg-binding protein" having a binding activity towards Reg protein can be prepared using a known hybridization technique (Sambrook, J. et al. (1989) Molecular Cloning 2nd ed., Cold Spring Harbor Laboratory Press) and polymerase chain reaction (PCR) technique (Sambrook, J. et al. (1989) Molecular Cloning 2nd ed., Cold Spring Harbor Laboratory Press; Innis, M.A. et al., PCR Protocols, Academic Press (1990)). Namely, it is routine for one skilled in the art to isolate a DNA highly homologous to rat "Reg-binding protein" cDNA from various other organisms using the rat "Reg-binding protein" cDNA (SEQ ID NO: 1 or 3), or portions thereof, as probe, and oligonucleotides specifically hybridizing to the rat "Reg-binding protein" cDNA as primer, to obtain proteins structurally similar to the rat "Reg-binding protein" from the isolated DNA.

A protein encoded by DNA hybridizing to the rat "Reg-binding protein" cDNA is included in this invention, as long as it has a binding activity towards rat "Reg-binding protein". Other organisms used for isolating such a protein include, for example, humans, monkeys, mice, rabbits, goats, cattle, pigs, dogs and so on, but are not limited thereto. β cells of pancreatic Langerhans' islet of these organisms are thought to be a suitable source when isolating DNA encoding such a protein.

DNAs encoding the "Reg-binding protein" derived from organisms other than rats are usually highly homologous to the cDNA sequence (SEQ ID NO: 1 or 3) of rat "Reg-binding protein". "Highly homologous" means at least 60% or more, preferably 80% or more, and more preferably 90% or more, even more preferably 95% or more, most preferably 99% or more sequence identity at the nucleotide sequence level. The homology of the sequence can be determined by FASTA (searches one with wide range sequence similarity) , BLAST (searches one with locally high similarity) and SSEARCH (search employing Smith-Waterman algorithm). These can be used by going to well-known databases and websites such as DNA Data Bank of Japan (DDBJ).

Hybridization conditions for isolating, from an organism other than the rat, a cDNA encoding a protein functionally equivalent to rat "Reg-binding protein" using rat "Reg-binding protein" cDNA, can be suitably selected by one skilled in the art. For example, hybridization can be carried out at 42°C using 6x SSC, 5x FBP, 0.5% SDS, 0.2 mg/ml salmon (herring) sperm DNA, and 10% formamide solution (low-stringent conditions). Preferably, the hybridization is carried out at 42°C using 6x SSC, 5x FBP, 0.5% SDS, 0.2 mg/ml salmon (herring) sperm DNA, and 30% formamide solution (medium-stringent conditions). More preferably, the hybridization is carried out at 50°C using 6x SSC, 5x FBP, 0.5% SDS, 0.2 mg/ml salmon (herring) sperm DNA, and 50% formamide solution (highly-stringent conditions). In this case, although several factors including temperature, formamide concentration, salt concentration, and such are thought to influence the stringency of hybridization, one skilled in the art can accomplish similar stringencies by suitably selecting these factors.

The protein of this invention can be prepared as either a natural protein or a recombinant protein utilizing gene recombination techniques. A natural protein can be prepared by, for example, subjecting extracts from tissues that are thought to express the "Reg-binding protein" (for example, β cells of pancreatic Langerhans' islet) to affinity chromatography using an antibody against the "Reg-binding protein" as described below. On the other hand, a recombinant protein can be prepared by culturing cells transformed with DNA encoding the "Reg-binding protein", allowing the transformants to express the protein, and recovering the protein as described below.

The present invention includes partial peptides of the protein of the present invention. An example of partial peptides of the proteins of the present invention is a peptide corresponding to the Reg protein-binding site. By administering a partial peptide of the present invention to a living body, it can be utilized as an agonist or antagonist of the protein of the present invention, or an antagonist, and such, of the Reg protein. Such partial peptides are useful as activators or inhibitors of signal transduction mediated by the protein of this invention. Additionally, the partial peptides of this invention include a partial peptide of the N-terminal region, or the C-terminal region of the protein of this invention, and these peptides can be utilized to prepare antibodies. Partial polypeptides comprising amino acid sequences specific to the protein of this invention have at least 7, preferably at least 8, more preferably at least 9 amino acid residues. Partial peptides of this invention can be produced by, for example, genetic engineering techniques, known peptide synthesizing methods, or by cleaving the protein of this invention with appropriate peptidases. For example, partial peptides comprising domains binding to Reg protein can be used for binding to Reg protein. Such partial peptides can be used as Reg protein-binding agents.

This invention relates to DNAs encoding the protein of the invention. DNA encoding the protein of this invention is not particularly limited as long as it can encode the protein of this invention, and includes cDNA, genomic DNA, and synthetic DNA. DNA having any nucleotide sequence based on the degeneracy of genetic codes is also included in this invention as long as they can encode the protein of this invention.

cDNA encoding the protein of this invention can be screened, for example, by labeling cDNA of SEQ ID NO: 1 or 3 or fragments thereof, RNA complementary to them, or synthetic oligonucleotides comprising partial sequences of the cDNA with ³²P and such, and hybridizing them to a cDNA library derived from tissues (e.g., pancreas, etc.) expressing the protein of this invention. Also, such cDNAs can be cloned by synthesizing oligonucleotides corresponding to nucleotide sequences of the cDNAs, and amplifying them by polymerase chain reaction with cDNA derived from suitable tissues (e.g. pancreas, etc.) as a template. Genomic DNA can be screened, for example, by labeling cDNA of SEQ ID NO: 1 or 3 or segments thereof, RNA complementary to them, or synthetic oligonucleotides comprising partial sequences of the cDNA with ³²P and such, and hybridizing them with a genomic DNA library. Alternatively, the genomic DNA can be cloned by synthesizing oligonucleotides corresponding to nucleotide sequences of these cDNAs, and amplifying them by polymerase chain reaction using genomic DNA as a template. On the other hand, synthetic DNAs can be prepared, for example, by chemically synthesizing oligonucleotides comprising partial sequences of cDNA of SEQ ID NO: 1 or 3, annealing them to form a double strand, and ligating them by DNA ligase.

These DNAs are useful for the production of recombinant proteins. Namely, the protein of the present invention can be prepared as a recombinant protein by inserting DNAs encoding the protein of this invention (e.g. SEQ ID NO: 1 or 3) into an appropriate expression vector, transforming suitable cells with the vector, culturing the transformants, and recovering the expressed protein. The protein of the present invention can be prepared as a purified or crude protein, or in the membrane-bound form after expressing in mammalian cells.

Example of specific host-vector systems are, *E. coli*-pGEX system (Amersham Pharmacia Biotech; expressed as GST-fusion protein), *E. coli*-pHB6 system and pVB6 system (Roche diagnostics; expressed as 6xHis-fusion protein), *E. coli*-pMAL system (New England Biolabs; expressed as a fusion protein with maltose-binding protein), *E*. *coli*-pTYB system (New England Biolabs; expressed as a fusion protein with Intein (Intein part is digested under the presence of DTT facilitating purification of only the objective protein) , Pichia-pPIC system and pGAP system (Invitrogen), mammalian cells (for example, COS7)-pCI-neo system (Promega) and pHook system (Invitrogen), and such.

Vectors can be introduced into hosts by the well known transformation into competent cells or electroporation for *E. coli*, transformation into competent cells prepared with Pichia Easy Comp kit (refer to Example 1) or electroporation for *Pichia,* electroporation or well known lipofection method using cationic lipids for mammalian cells, etc.

Recombinant proteins expressed in host cells can be purified by known methods. The protein of this invention expressed in the form of a fusion protein, for example, with a histidine residue tag or glutathione-S-transferase (GST) attached at the N-terminus can be purified by a nickel column or a glutathione sepharose column, etc.

DNA encoding the protein of the present invention can also be applied to gene therapy against diseases caused by a mutation therein. For example, gene therapy using a vector of a virus such as the vaccinia virus or retrovirus can be given. An actual therapeutic method would be: introducing "Reg-binding protein" into, for example, pancreas or the Langerhans' islets to be used in a transplantation, under culture conditions using these recombinant viruses, and conducting transplantation. This would improve the therapeutic effects of the transplantation through the proliferation of pancreatic β cells, and enable effective use of the transplanting organ.

The present invention also relates to a polynucleotide comprising at least 15 nucleotides hybridizing to DNA comprising the nucleotide sequence described in SEQ ID NO: 1 or SEQ ID NO: 3, or complimentary DNA thereof. The polynucleotide preferably hybridizes specifically to DNA comprising the nucleotide sequence described in SEQ ID NO: 1 or SEQ ID NO: 3, and comprises at least 15 nucleotides. "Hybridize specifically" means that no significant cross-hybridization with DNA encoding other proteins is observed under the normal hybridization conditions, preferably under the medium-stringent hybridization conditions described above, more preferably under the highly stringent hybridization conditions described above. Hybridization can be conducted at the conditions described above. These polynucleotides include probes and primers, nucleotides or nucleotide derivatives (for example, antisense oligonucleotides and ribozymes), which can specifically hybridize to DNA encoding the protein of the present invention or the DNA complementary to the DNA.

Oligonucleotides comprising cDNA encoding the protein of the invention or a partial sequence thereof can be used for the cloning of genes or cDNA encoding the protein of the present invention or the amplification by PCR. Moreover, they are useful for the detection and quantification of RNA encoding the protein of the present invention. Furthermore, they can be used for detecting a mutation, polymorphism, or disorder (such as gene diagnosis), by methods such as restriction fragment length polymorphism (RFLP), single strand conformation polymorphism (SSCP).

The polynucleotide of the present invention can be used for pancreatic tests, for example a pancreatic β cell test, since the protein of the present invention has important functions in the formation, regeneration and/or maintenance of the pancreas, especially in the regulation of pancreatic β cell mass. Moreover, the polynucleotide of the present invention can be used in diabetes tests. For example, pancreatic tissue samples are isolated from subjects and abnormalities in the expression levels of the protein of the present invention in these tissues can be examined by methods such as northern hybridization, RT-PCR, or DNA chip (DNA microarray). Moreover, the presence or absence of a mutation or polymorphism of the DNA or RNA encoding the protein of the present invention can be tested by sequence analysis, SSCP, RFLP, etc. In the case of using the polynucleotide as a test reagent, it can be properly mixed with distilled water, a buffer, salt, and so on.

Moreover, the protein of the present invention or partial peptides thereof, DNA encoding the protein or peptides, and vectors into which the DNA has been inserted can be used for the below-mentioned screening of compounds inhibiting the binding of the protein of the present invention and Reg protein. It can also be used for screening compounds promoting or inhibiting the signal transduction (for example, cell growth activity or DNA-synthesizing activity of cells) stimulated by the activation of the protein of the present invention. These screenings can be applied for assaying therapeutic agents or preventive drugs for diseases caused by disorders in the mass or functions of pancreatic β cells, including diabetes. The screenings can also be used for assaying or screening therapeutic agents or preventive drugs for gastrointestinal tumors, neurodegeneration diseases, pancreatitis, and other tumors, besides diabetes.

Moreover, the present invention relates to an antibody binding to the protein of the present invention. The antibody of the present invention includes polyclonal and monoclonal antibodies. A polyclonal antibody can be prepared by immunizing a rabbit, goat, sheep, or such by a well known method (Harlow, E. and Lane, D. Antibodies, Cold Spring Harbor Laboratory (1988), etc.) using as the antigen a "Reg-binding protein" prepared from a biomaterial (for example, pancreas Langerhans' islet), a recombinant "Reg-binding protein" produced by a host-vector system, and such described above, or partial peptides synthesized by ordinary peptide synthesis methods. A monoclonal antibody can be prepared by immunizing a mouse, rat, or such, by a well known method (Harlow, E. and Lane, D. Antibodies, Cold Spring Harbor Laboratory (1988), etc.) using as the antigen a "Reg-binding protein" prepared from a biomaterial (for example, pancreas Langerhans' islet), a recombinant "Reg-binding protein" produced by a host-vector system, and such described above, or partial peptides synthesized by ordinary peptide synthesis methods, and using splenocytes of the mouse, rat, or such, to obtain a hybridoma which produces the monoclonal antibody.

Antibodies are purified by ordinary biochemical methods such as ammonium sulfate fractionation, protein G Sepharose column, or affinity columns in which an antigen is immobilized, from serum in the case of polyclonal antibodies, and from the culture supernatant of hybridoma or ascites of animals inoculated with the hybridoma in the case of monoclonal antibodies.

Antibodies thus prepared are used for the affinity purification of the proteins of this invention or, can be used for testing and diagnosing disorders caused by abnormal expression or structural abnormalities of the protein of this invention and for detecting the expression level of the protein, etc. Specifically, for example, proteins are extracted from tissues or cells, and through the detection of protein of the present invention by Western blotting, immunoprecipitation, ELISA, and such, abnormalities in the expression or structure can be tested and/or diagnosed. The antibody of the present invention can be also used for pancreatic tests, for example, pancreatic β cell tests. Moreover, the antibody of the present invention can be used for testing diabetes. For example, by isolating a pancreatic tissue sample from a subject, abnormalities in the expression level or structure of the protein of the present invention in the tissue can be tested by Western blotting, immunohistochemistry, ELISA, EIA, and such. In the case of using the antibody as a test reagent, sterilized water, buffer, salt, stabilizer, preservative, and such can be combined appropriately. Moreover, the antibody of the present invention may also be used for antibody therapy. In the case of using the antibody of the present invention for antibody therapy, humanized or human antibodies are preferable. In this case, human lymphocytes and HGPRT (hypoxanthine-guanine phosphoribosyl transferase)-deficient myeloma cells are fused and human-mouse heterohybridomas are selected using HAT medium. Myeloma cells are selected by the well-known RIA or ELISA method in which "Reg-binding protein" is used as the antigen, and clones producing humanized monoclonal antibody are obtained. Purification of the antibody can be conducted as described above.

The present invention also relates to a method for screening a compound binding to the protein of this invention. Such a screening can be carried out by a method comprising the following steps: (a) contacting the protein of the invention or its peptide with a test sample, (b) detecting the binding of the test sample to the protein of the invention or its peptide, and, (c) selecting a compound that binds to the protein of the invention or its peptide.

The protein of the present invention can be used for the screening as a purified protein, in the cell surface-expressed form, or as a cell membrane fraction, according to the method of screening.

Test samples, for example, cell extracts, expression products of gene libraries, synthetic low molecular weight compounds, synthetic peptides, natural compounds, and such, can be used, but are not limited thereto. The test samples used for screening can be labeled prior to use as necessary. Labels include, for example, radioactive and fluorescent labels, and such, but are not limited thereto.

Screening of a protein binding to the protein of the present invention can be carried out, for example, by applying the culture supernatant of cells, or cell extract expected to express proteins binding to the protein of this invention, to an affinity column in which the protein of this invention has been immobilized, and by purifying a protein that specifically binds to this column.

Moreover, it can be conducted according to "West-Western blotting method", and such, in which a cDNA library is constructed from tissues or cells (for example, pancreatic β cells) expected to express the protein binding to the protein of the present invention, and then, this is expressed on agarose and the protein expressed is immobilized on the filter and reacted with labeled protein of the present invention to detect plaques expressing the binding protein. Another method is the "two-hybrid system" in which GAL4-DNA binding domain and GAL4 transcriptional activation domain are expressed as a fusion protein of the present invention and the test protein, and the binding of the protein of the present invention and the test protein is detected through the expression of a reporter gene linked to the downstream of a promoter with the binding sequence of GAL4-DNA binding protein.

Moreover, the method in which the immobilized protein of the present invention is reacted with a synthetic compound, natural product bank, or a random phage peptide display library to screen the binding protein, and the method in which a compound binding to the protein of the present invention is isolated by screening by combinatorial chemistry techniques using high-throughput system, are techniques well known to one skilled in the art.

Moreover, a screening using BIACORE (Biacore), or a method in which changes in acid secretion speed of cultured cells forced to express Reg-binding protein of the present invention are monitored by using a microphysiometer (Molecular Device) , and such, can be given as examples.

Moreover, the present invention relates to a method for screening a compound that inhibits the binding of the protein of the present invention and Reg protein. Such a screening can be conducted by a method including the following steps of: (a) contacting Reg protein with the protein of the present invention in the presence of a test sample, (b) detecting the binding of Reg protein to the protein of the present invention, and, (c) selecting a compound that decreases the binding.

The protein of the present invention can be used for the screening as a purified protein, in the cell surface-expressed form, or as a cell membrane fraction. Reg protein is usually used for screening as a purified protein. For example, human REG Iα or rat Reg I, and such can be used as the Reg protein. These proteins can be prepared as recombinant proteins (refer to Example 1). Reg protein can be labeled with radioisotopes such as [¹²⁵I], if necessary.

As test samples, for example, cell extract solutions, expression products of gene libraries, synthesized low molecular compounds, synthesized peptides, natural compounds, and such can be used, but are not limited thereto.

Screening can be conducted, for example, as follows. Cells expressing the protein of the present invention or a membrane fraction prepared using them are contacted with a labeled ligand (Reg protein) under the presence of a test sample, and the amount of the labeled ligand binding to the protein of the present invention is measured. A compound that lowers the amount of ligand as compared with the case in the absence of the test sample is selected. The binding of the protein of the present invention and Reg protein can be measured using BIACORE or microphysiometer described above. Compounds thus isolated can be the candidates for antagonists or agonists of the protein of the present invention.

Moreover, the present invention relates to a method for screening a compound that promotes or inhibits the signal transduction caused by the activation of the protein of the present invention. Such a screening can be conducted by the following steps: (a) contacting Reg protein with a cell expressing the protein of the present invention on the cell surface in the presence of a test sample, (b) detecting a change of the cell in response to the stimulation by Reg protein, (c) selecting a compound that enhances or suppresses the change of the cell as compared to when detected in the absence of the test sample (control).

Cells expressing the protein of the present invention on its surface can be prepared by inserting DNA encoding the protein of the present invention into an appropriate expression vector and introducing it to appropriate host cells. For example, cells such as RINm5F cells, CHO cells, COS-7 cells can be given as host cells. As for the vector, pCI-neo (Promega), pHook (Invitrogen) and such can be given.

As test samples, for example, cell extracts, expression products of the gene libraries, synthesized low molecular compounds, synthesized peptides, natural compounds and such are used, but are not limited thereto. Moreover, as a test sample, it is also possible to use a compound isolated by the above-described screenings using as an index the binding with the protein of the present invention.

Reg protein is used for screening usually as a purified protein. As the Reg protein, for example, human REG Iα or rat Reg I, and such can be used. These proteins can be prepared as recombinant proteins (refer to Example 1).

In the screening, a change of the cells described above in response to the Reg protein stimulation under the presence of a test sample is detected. As the change of the cells in response to the Reg protein stimulation, for example, a change in cell growth activity, a change in DNA synthesis activity, a change in the degree of apoptosis of cells, phosphorylation of the protein of the present invention or proteins transducing signals, a change in the expression of a specific gene in the cells, and such, can be given, but the change is not limited thereto.

The DNA synthesis of cells can be detected, for example, as indicated in examples, by measuring the incorporation of 5'-bromo-2'-deoxyuridine (BrdU). Moreover, the detection can be conducted by measuring radioactivity incorporated after the addition of ³H-thymidine to cells. The test of ³H-thymidine incorporation to cells is generally used to assay the promotion or inhibition effect on DNA synthesis. The method has the advantages of enabling the handling of a relatively large amount of samples, with a high sensitivity, etc. In the screening of a compound promoting or inhibiting DNA synthesis, specifically, for example, cells are seeded onto a multi-well plate and such, and after 1-2 day incubation, medium is changed to a medium containing the test sample and incubated for certain duration such as 24 hours. Thereafter, for example, 1 µCi/ml of ³H thymidine is added. After incubating, the medium is removed, washed, 10% TCA is added, and then, the cells are left to stand for approximately 20 min, and washed with ice cold 5% TCA. The cells are then lysed with 0.5 N NaOH, left to stand on ice for 10 min, 1/2 volume of 1 N HCl is added and gently mixed, then, 40% TCA is added to a final concentration of 10%, and gently mixed. After standing on ice for 20 min, the solution is filtrated by a Whatman GF/C filter, and such, to collect insoluble material. After washing with 100% ethanol for 3 times and drying, radioactivity is measured using a liquid scintillation counter.

Moreover, the cell growth can be measured by measuring cell numbers or colony numbers, or by measuring a color development that is dependent on the cell number by adding dyes such as MTT or Alamar Blue. The MTT method measures cell growth activity using color development by MTT (3-(4,5-dimethylthiazol-2-yl)-2,5-diphenyltetrazolium bromide), and MTT formazan is formed due to a reaction with the respiratory chain of mitochondria of living cells. The amount produced reflects the cell number. Specifically, for example, cells are incubated in a 96-well plate, reacted with a test sample, and then, 10 µl of 5 mg/ml MTT solution is added, and incubated for 4 hours. Then, 100 µl of 0.04 N HCl/isopropanol is added, mixed well, and left to stand for several minutes. Then, the coloring is measured using a microplate reader at the reference wavelength of 630 nm and test wavelength of 570 nm. Moreover, as described in Example 11, tetrazolium salt 4[-3-(4-lodophenyl)-2-(4-nitrophenyl) -2H-5-tetrazolyo]-1,3-benzenedisulfonate (WST-1) can be used for the assay.

The apoptosis of cells can be assayed, for example, using morphological changes in the nucleus (condensation or segmentation of nucleus), fragmentation of chromosomes (ladder formation) and such, as indexes. Specifically, apoptosis can be detected, for example, by the TUNEL method (Y. Gavrieli et al., J. Cell Biol. 119, 493(1992)), and so on (refer to Example 11).

Protein phosphorylation is considered to occur in serine, threonine or tyrosine residues. These changes of phosphorylation can be detected by measuring the phosphorylation state of intracellular proteins by Western blotting method or immunoprecipitation method using anti-phosphoserine, anti-phosphothreonine, or anti-phosphotyrosine antibodies. Cell proliferation-related proteins such as MAP kinase family, STAT family, or Fos-Jun family protein can be expected to be phosphorylated, but are not limited thereto.

It is known that transcription of various genes is induced or suppressed by the protein phosphorylation described above, etc. Changes in expression of a specific gene depending on the binding of the protein of the present invention and its ligand can be detected using a reporter gene. Namely, the change in expression can be measured by detecting reporter gene expression in which the reporter gene is linked to the downstream of the promoter of the gene. Moreover, a change in expression of a specific gene can also be measured by northern blotting or RT-PCR method in which mRNA is detected, a method using an antibody to detect proteins that are gene translation products, or a method detecting the activity of proteins that are gene translation products.

Compounds isolated by these screenings include, for example, (1) compounds that bind to the protein of the present invention and promote or inhibit its activity, (2) compounds that bind to the protein of the present invention, or ligands of the protein of the present invention like Reg protein or the like, and promote or inhibit the binding of the protein of the present invention and ligands, (3) compounds that bind to ligands of the protein of the present invention and promote or inhibit their activation, and (4) compounds that promote or inhibit the signal transduction from the protein of the present invention to the expression of a changes of cells.

Such compounds can be applied as preventive or therapeutic agents against diseases caused by disorders of signal transduction systems that mediate the protein of the present invention (for example, diseases caused by functional disorders of pancreatic β cells). For example, these compounds can be applied as therapeutic agents for diabetes.

DNAs of the present invention, proteins of the present invention or partial peptides thereof, vectors comprising DNAs of the present invention, antibodies against the protein of the present invention or partial peptides thereof, and compounds isolated by the screenings described above, can be used alone, or as a combination with other compounds when using as therapeutic agents. Reagents and drugs are included in the therapeutic agent of the present invention.

For example, since the protein of the present invention has a binding activity towards Reg protein, the protein of the present invention and partial peptides thereof can be used for the binding to Reg protein. Such proteins or peptides can be used for the detection of the Reg protein or for affinity purification. By contacting the protein of the present invention or partial peptides thereof with the Reg protein, the protein of the present invention or partial peptides thereof can be bound to the Reg protein. The protein of the present invention or partial peptides thereof may have been purified or expressed on the cell membrane surface. They can also be bound to carriers. There is no limitation on the origin of the Reg protein to be bound, and the mouse, rat, or human Reg protein can be used. Moreover, DNAs encoding the protein of the present invention or partial peptides thereof, and vectors to which the DNAs have been inserted can be used for the same purpose by expressing the protein of the present invention or partial peptides thereof in the cells. Thus, the protein of the present invention or partial peptides thereof, DNAs encoding them, or therapeutic agents comprising vectors carrying the DNAs can be Reg protein-binding agents.

Moreover, the protein of the present invention functions as a Reg protein receptor. Therefore, the protein of the present invention can be used for the regulation (promotion or suppression) of intracellular signal transduction in response to the Reg protein. By activating the protein of the present invention, the signal transduction is promoted, and inversely, by inhibiting the activation, signal transduction is blocked. For example, by contacting cells expressing the protein of the present invention (for example, SEQ ID NO: 4) with ligands of the protein of the present invention such as Reg protein, or agonists, the protein of the present invention is activated and signals are transduced to the cell interior. Cells are preferably of pancreatic β cell lineage, epithelial cells, etc. Moreover, proteins that bind to Reg protein, but do not transduce signals to cell interior, can be used for blocking the signal transduction of the Reg protein. As an example, a protein comprising the region binding to Reg protein, but not the region that transduces signals to the downstream can be given. By expressing such proteins in the cells, or adding them extracellularly, the signal transduction by Reg protein can be blocked. DNAs encoding the protein of the present invention or partial peptides thereof, and vectors to which the DNAs have been inserted can be used for the same purpose, by expressing the protein of the present invention or partial peptides thereof in the cells. Moreover, antibodies binding to the protein of the present invention or partial peptides thereof, or compounds isolated by the screenings of the present invention can be used for the same purpose. Therefore, the protein of the present invention or partial peptides thereof, DNAs encoding the proteins or peptides, vectors to which the DNAs have been inserted, antibodies of the present invention, and compounds isolated by the screening of the present invention, can be regarded as regulators (promoters, suppressors, etc.) of intracellular signal transduction in response to Reg protein.

Examples of the intracellular signal transduction in response to Reg protein are, promotion of cellular DNA synthesis and regulation of cell growth (promotion or suppression). Namely, this shows that the protein of the present invention can be used for suppressing cellular DNA synthesis, and promotion or suppression of cell growth. Target cells are preferably cells of pancreatic β cell lineage, epithelial cells, etc. Cell growth (or cell division) can be promoted by contacting cells expressing the protein of the present invention with ligands (for example, Reg protein) or agonists of the protein of the present invention to promote DNA synthesis. When expressing the protein of the present invention exogenously in cells, vectors expressing the protein of the present invention (for example, SEQ ID NO: 4) are introduced into the cells. Moreover, proteins that bind to Reg protein, but do not transduce signals to the cell interior can be used for inhibiting DNA synthesis or cell growth. As an example, a protein comprising the region binding to Reg protein, but not the region that transduces signals to the downstream can be given. DNA synthesis or cell growth can be suppressed by expressing such proteins intracellularly or adding them extracellularly. DNAs encoding the protein of the present invention or partial peptides thereof, or vectors to which the DNAs have been inserted, can be used for the same purpose, by expressing the protein of the present invention or partial peptides thereof in the cells. Moreover, antibodies binding to the protein of the present invention or partial peptides thereof, and compounds isolated by the screening of the present invention can be used for the regulation of DNA synthesis or cell growth. For example, antibodies or compounds functioning as ligands or agonists of the protein of the present invention can promote growth of cells (such as pancreatic β cells), by administrating these ligands and agonists to the living body. The administration can be conducted *in vitro* and *in vivo.* Thus, the protein of the present invention or partial peptides thereof, DNAs encoding the protein or peptides, vectors to which the DNAs have been inserted, antibodies of the present invention, and compounds isolated by the screening of the present invention can be regulators (promoters or suppressors) of cellular DNA synthesis or cell growth.

Moreover, as an example of the signal transduction elicited by the activation of the protein of the present invention, cell apoptosis can be given. Namely, the protein of the present invention can be used for the regulation of cell apoptosis (induction of apoptosis or the suppression of the induction). DNAs encoding the protein of the present invention or partial peptides thereof, and vectors into which the DNAs have been inserted can be used for the same purpose, by expressing the protein of the present invention or partial peptides thereof in the cells. Moreover, antibodies binding to the protein of the present invention or partial peptides thereof, and compounds isolated by the screening of the present invention can also be used for the regulation of apoptosis. Target cells are preferably of pancreatic β cell lineage, epithelial cells, etc. Apoptosis can be induced by contacting cells expressing a high concentration of the protein of the present invention with ligands of the protein of the present invention (for example, Reg protein). Reg protein is contacted with cells at a concentration higher than 100 nM, preferably 500 nM or more, more preferably 1000 nM or more. In the case of expressing the protein of the present invention exogenously in the cells, vectors expressing the protein of the present invention (for example, SEQ ID NO: 4) are introduced into the cells. Moreover, proteins that bind to Reg protein, but do not transduce signals to the cell interior can be used to suppress apoptosis caused by Reg protein. Apoptosis can be suppressed by expressing such proteins intracellularly or by adding them extracellularly. Thus, the protein of the present invention and partial peptides thereof, DNAs encoding the protein or partial peptides thereof, vectors into which the DNAs have been inserted, antibodies of the present invention, and compounds isolated by the screenings of the present invention can be regulators (inducers or suppressors etc.) of apoptosis of cells.

The protein of the present invention or partial peptides thereof, DNAs encoding the protein or peptides, vectors into which the DNAs have been inserted, antibodies of the present invention, and compounds isolated by the screenings of the present invention can be made into a composition by combining with distilled water, a salt, BSA, glycerol, a stabilizer, preservative, or detergent, according to well known pharmacological methods. Moreover, the pharmaceutical agent of the present invention can be used as a reagent for pancreatic tests as described above. Moreover, it is also useful as a pharmaceutical composition for treating or preventing diabetes, digestive tract tumors, neurodegeneration diseases, pancreatitis, and other tumors.

When using the pharmaceutical agent of the present invention as a drug, the protein of the present invention or partial peptides thereof, DNAs encoding the protein or peptides, vectors into which the DNAs have been inserted, antibodies of the present invention, and compounds isolated by the screening of the present invention can be directly administered to patients, or can be formulated by a well known pharmaceutical method. For example, a pharmaceutically acceptable carrier or medium, specifically, distilled water, physiological saline, dextrose, glycerol, ethanol, vegetable oil, an emulsifying agent, suspension, detergent, stabilizer, and such can be suitably combined for formulation and administered. The pharmaceutical composition of the present invention can be in the form of a solution, tablet, capsule, troche, buccal tablet, elixir, suspension, or syrup. The content of the active compound can be suitably determined. The administration can be conducted, for example, intranasally, transbronchially, intramuscularly, or orally by methods well known to one skilled in the art, in addition to intraarterial, intravenous, or hypodermic injections. Administration can be conducted systemically or topically. Dosage changes according to the weight, age of the patient, administration method, symptoms, and such, but a suitable dosage can be appropriately selected by one skilled in the art. Administration can be conducted once or several times. Moreover, as long as the compounds are materials encoded by DNA, gene therapy can be conducted by integrating the DNA into gene therapy vectors. Administration can be conducted *ex vivo* or *in vivo.* The administration method changes according to the weight, age, symptoms, and such, of the patient, but it can be appropriately selected by one skilled in the art.

### Brief Description of the Drawings

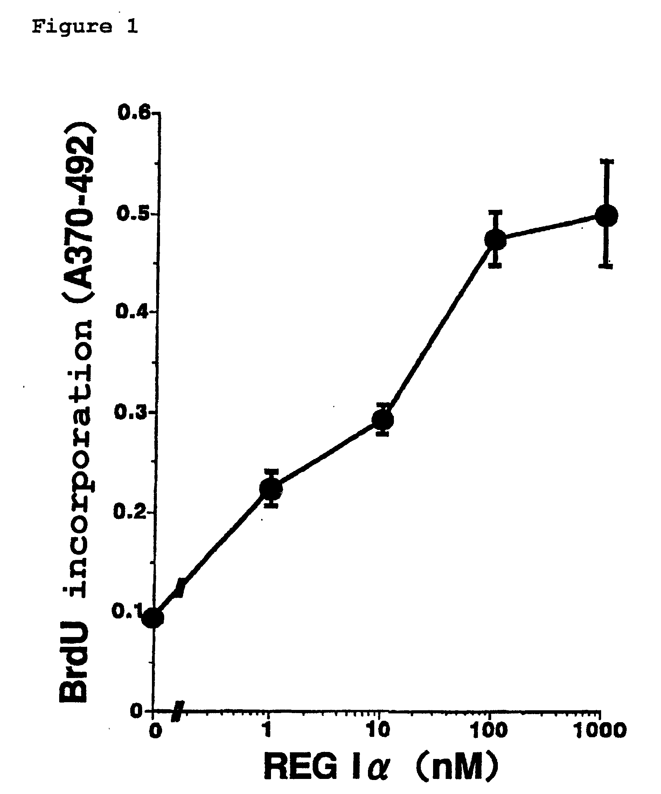

Figure 1 shows the result of measuring BrdU incorporation after the addition of human REG protein (REG Iα) to rat insulinoma derived cell line RINm5F cells (Example 3).

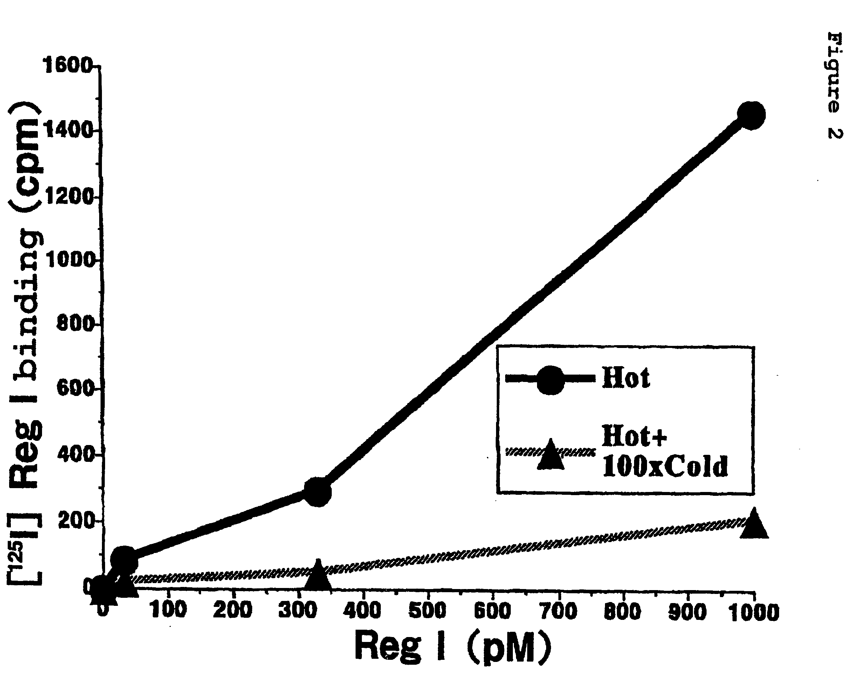

Figure 2 shows the result of measuring binding of [¹²⁵I] labeled rat Reg protein (Reg I) to RINm5F cells when it is added to the cells (Example 4). "Hot" indicates when only the labeled rat Reg protein is added, and "Hot + 100X Cold" indicates when both labeled rat Reg protein and 100-folds of non-labeled rat Reg protein is added.

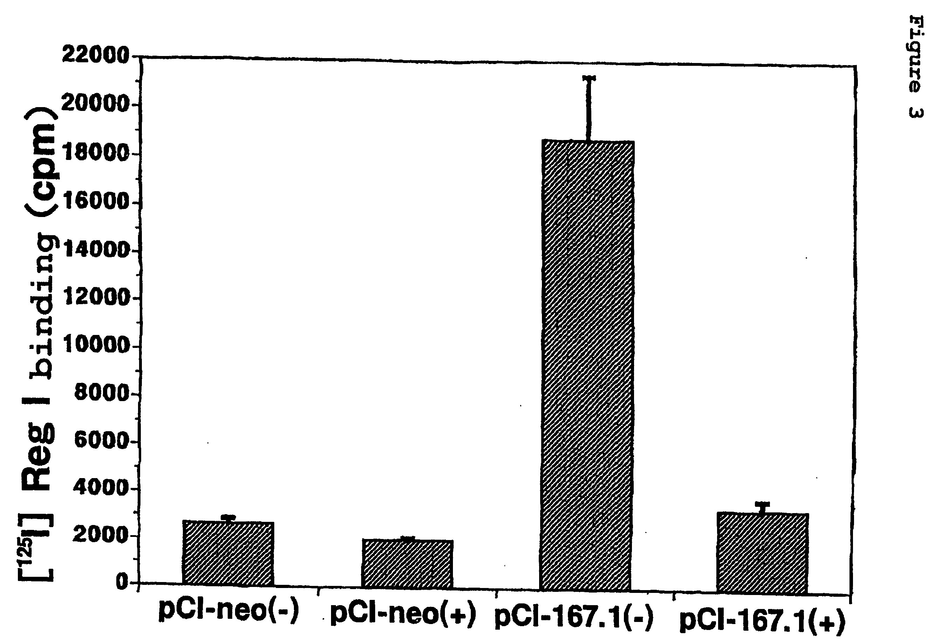

Figure 3 shows the result of measuring the binding of [¹²⁵I] labeled rat Reg protein (Reg I) to COS-7 cells expressed with isolated Reg-binding protein when [¹²⁵I] labeled rat Reg protein (Reg I) is added to the cells (Example 6). "pCI-neo" and "pCI-167.1" indicate results from cells introduced with empty vector and Reg-binding protein expression vector, respectively. Moreover, (-) and (+) indicate results in which labeled rat Reg protein only, and both labeled rat Reg protein and 100 times higher amount of non-labeled rat Reg protein are added, respectively.

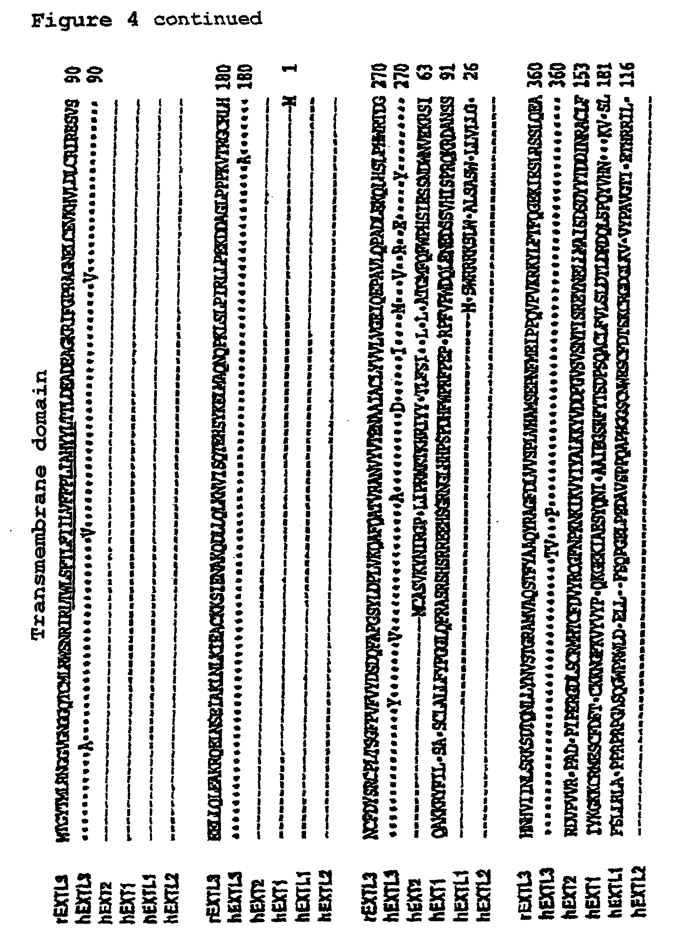

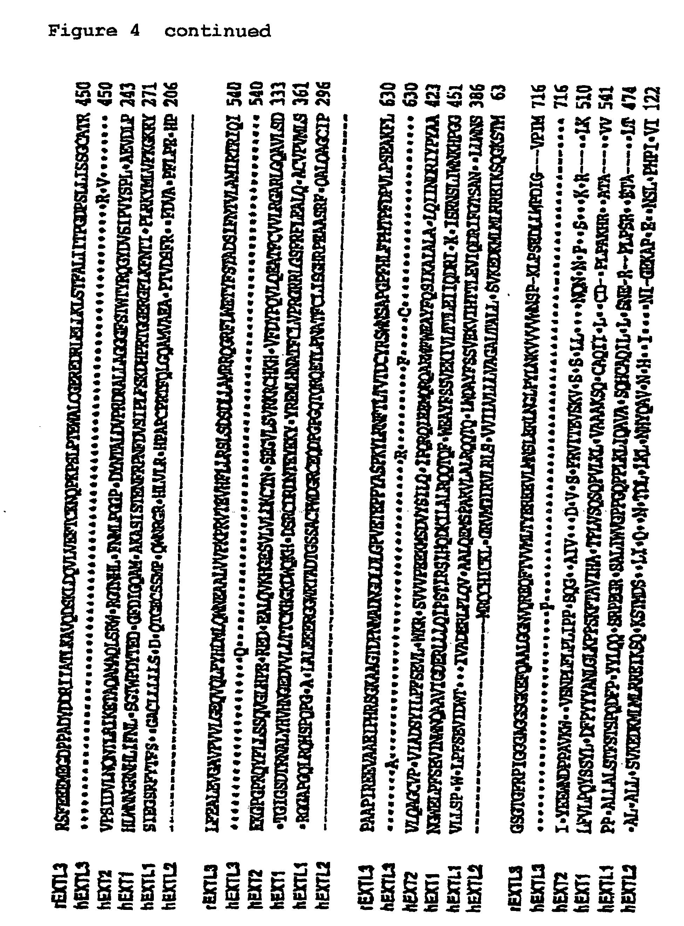

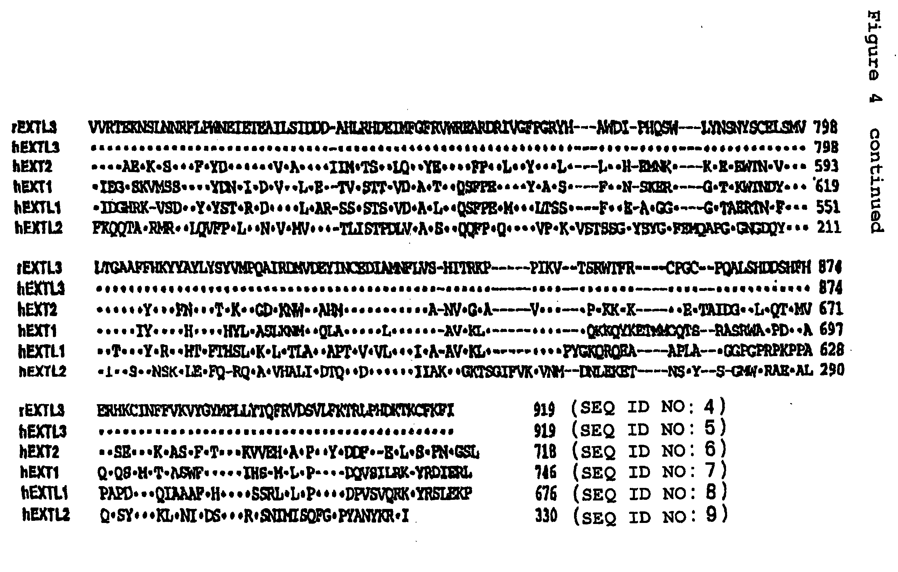

Figure 4 shows alignment of the predicted protein amino acid sequences of rat Reg receptor (rEXTL3)(SEQ ID NO: 4), human EXTL3/EXTR1 (hEXTL3) (GenBank accession numbers AF001690 and AB007042) (SEQ ID NO: 5), human EXT2 (hEXT2) (GenBank accession number U64511) (SEQ ID NO: 6), human EXT1 (hEXT1) (GenBank accession number S79639) (SEQ ID NO: 7), human EXTL1 (hEXTL1) (GenBank accession number U67191) (SEQ ID NO: 8), and human EXTL2 (hEXTL2) (GenBank accession number AF000416) (SEQ ID NO: 9) (Example 7). The transmembrane domain is underlined. The numbers on the right correspond to amino acid residues. Residues identical to rat Reg-binding protein (rEXTL3) are indicated by dots. Hyphens denote the absence of corresponding residues in rat Reg-binding protein (rEXTL3).

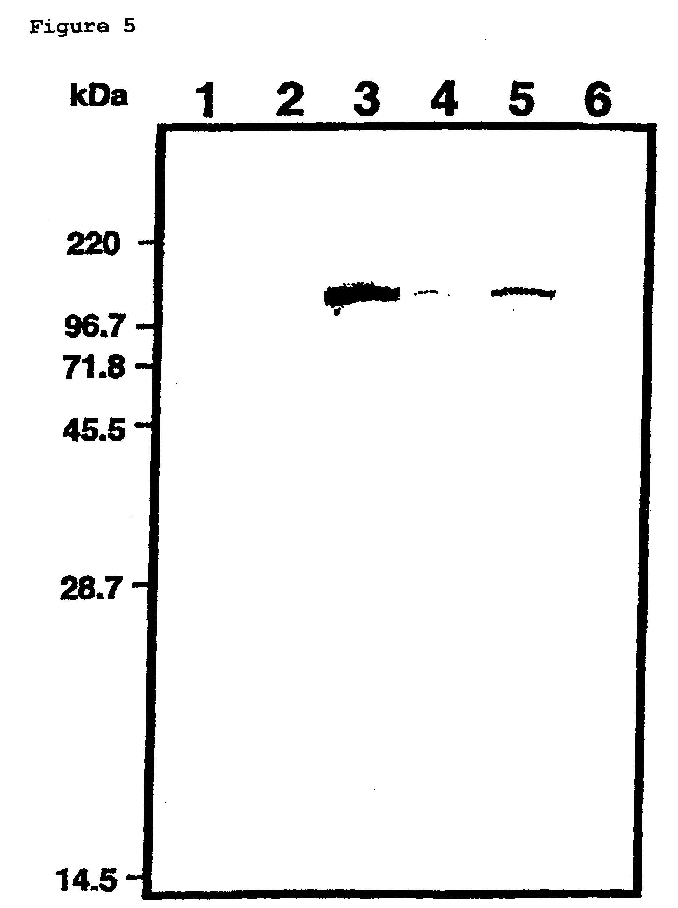

Figure 5 shows the cellular distribution of Reg-binding protein. Lane 1, homogenate of COS-7 cells to which the control vector had been introduced; lane 2-6, homogenate, membrane fraction, mitochondrial fraction, microsomal fraction and cytosolic fraction of COS-7 cell (Example 8) into which the Reg receptor expression vector had been introduced. Ten µg protein was electrophoresed in each lane, and Western blot analysis was carried out by using an antibody against the HA tag binding to Reg-binding protein.

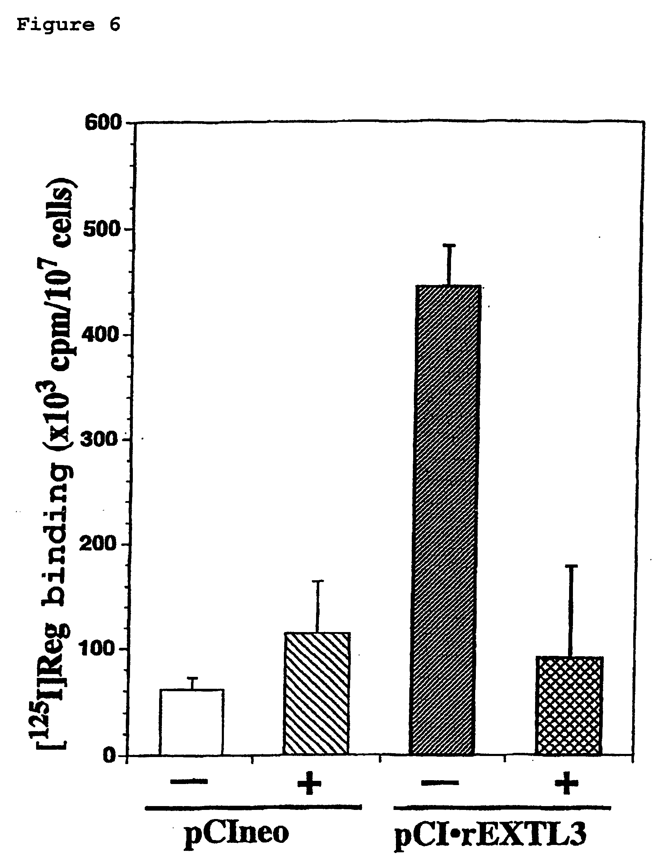

Figure 6 shows that the rat homologue of human EXTL3/EXTR1 is a cell surface type Reg-binding protein (Example 9). This figure shows the binding of [¹²⁵I] Reg protein to Reg receptor-expressing cells with (+; 100-fold excess) or without (-) unlabeled rat Reg protein. "pCIneo" is the control in which an empty vector has been introduced, and "pCI·rEXTL3" is the result of introducing Reg-binding protein expression vector to cells. Results are presented as the mean ± S.E.M. of 4 separate experiments.

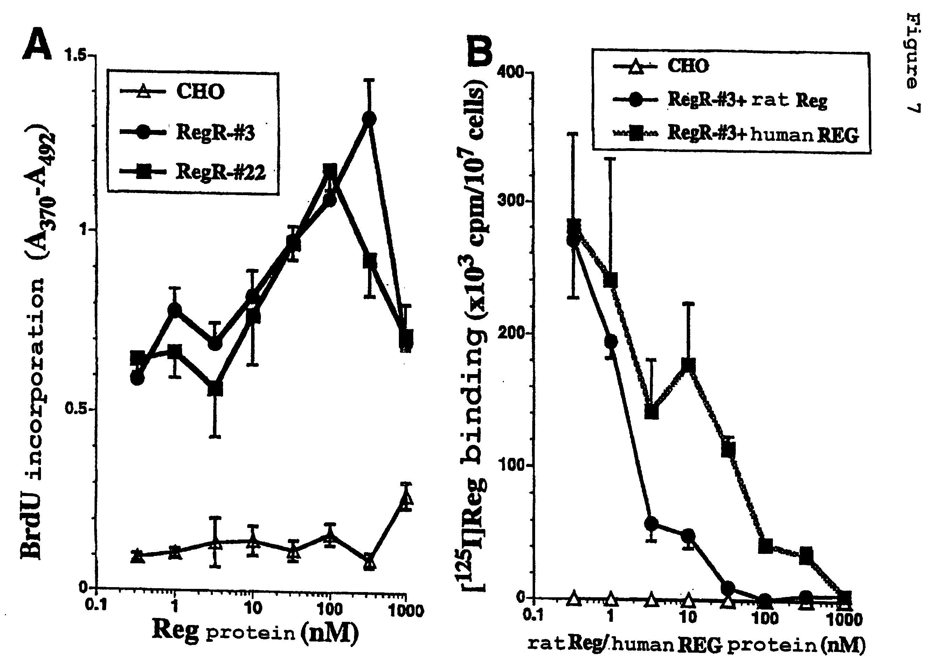

Figure 7 shows the functional characterization of Reg receptor.
   (A) BrdU incorporation by rat Reg protein into CHO cells stably expressing the Reg receptor (Example 10) . Two independent cell lines expressing the Reg receptor (RegR-#3 and RegR-#22) were tested. Results are presented as the mean ± S.E.M. of 8 separate experiments.
   (B) Competition binding curves for rat Reg (circle) and human REG (square) with rat Reg receptor. Results are presented as the mean ± S.E.M. of 4 separate experiments.

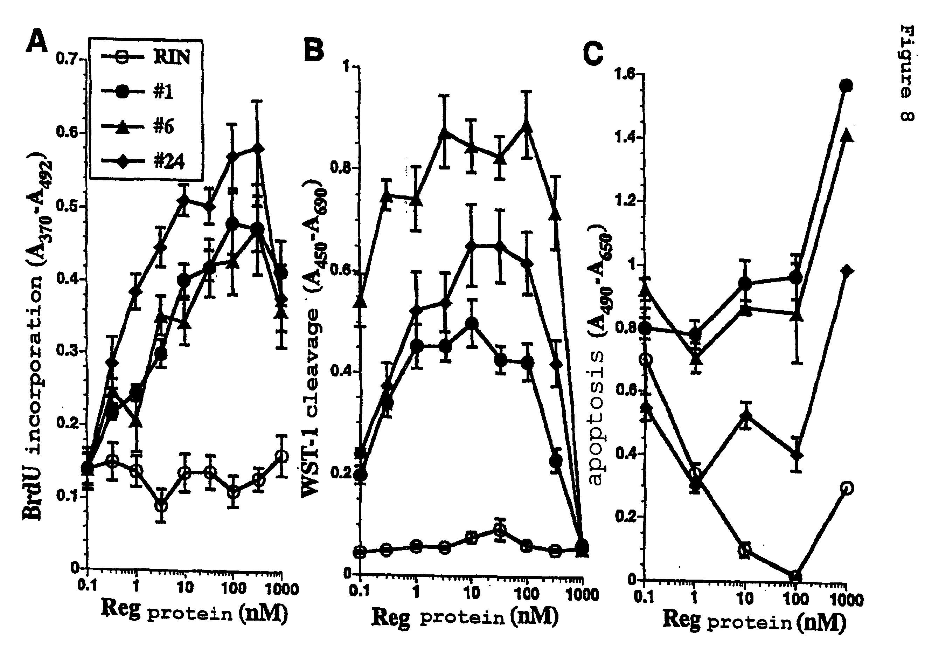

Figure 8 shows proliferation and apoptosis of Reg receptor expressing β-cells (Example 11). Three independent cell lines expressing the Reg receptor (#1, #6 and #24) were tested. RIN is the RINm5F control. Results are presented as the mean ± S.E.M. of 4-8 separate experiments. (A) BrdU incorporation by rat Reg protein into RINm5F cells stably expressing the Reg receptor. (B) Cleavage of WST-1 by viable cells was increased by Reg protein. (C) Reg protein-induced apoptosis of RINm5F cells was quantified by the TUNEL method.

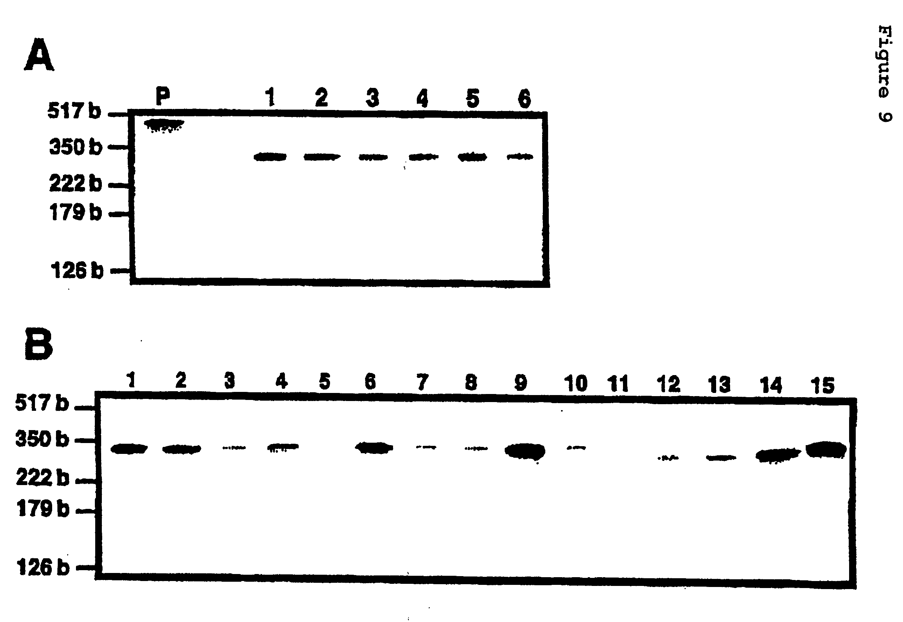

Figure 9 shows expression of Reg receptor mRNA (Example 12). RNase protection assay was carried out for measuring expression of Reg receptor mRNA. 309 nucleotide band corresponds to the protection size by Reg receptor mRNA. (A) Expression of Reg receptor mRNA in β-cells. Regenerating Langerhans' islets were isolated from 90% pancreatectomized rats receiving intraperitoneal administration of 0.5 mg/kg/day nicotinamide for 1-3 months (K. Terazono, et al., J. Biol. Chem. 263, 2111(1988); K. Terazono, T. Watanabe, Y. Yoneyama, in Molecular biology of the islets of Langerhans', H. Okamoto, Ed. (Cambridge University Press, Cambridge, 1990), pp. 301-313; K. Terazono et al., Diabetologia 33, 250(1990); Y. Yonemura et al., Diabetes 33, 401(1984)): Lane 1, normal pancreatic islets; lane 2, regenerating islets one month after the partial pancreatectomy: lane 3, regenerating Langerhans' islets two month after the partial pancreatectomy: lane 4, regenerating islets three month after the partial pancreatectomy: lane 5, RINmSF cells; lane 6, ARIP cells. Probe alone was applied in lane P. (B) Expression of Reg receptor mRNA in rat tissues: Lane 1, normal pancreatic islets; lane 2, whole pancreas; lane 3, liver; lane 4, kidney; lane 5, heart; lane 6, spleen; lane 7, thymus; lane 8, testis; lane 9, adrenal gland; lane 10, stomach; lane 11, jejunum; lane 12, ileum; lane 13, colon; lane 14, pituitary gland; lane 15, brain.

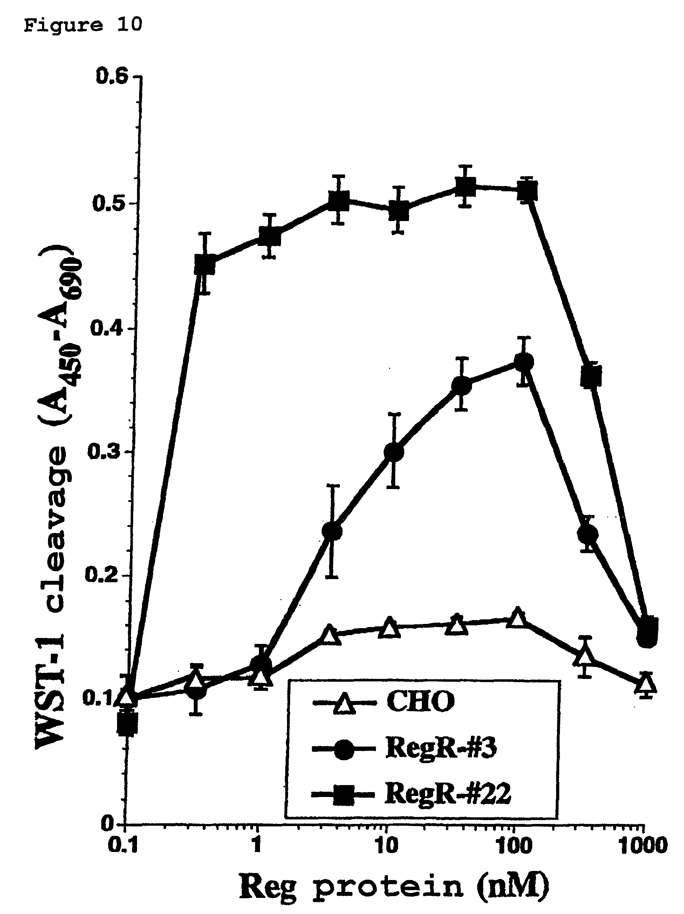

Figure 10 shows cleavage of WST-1 by viable cells increased by Reg protein in CHO cells stably expressing Reg receptor. Two independent cell lines as in Figure 7A were used. Results are presented as the mean ± S.E.M. of 8 separate experiments.

### Best Mode for Carrying out the Invention

Herein below, the present invention is explained specifically using examples, but it is not to be construed as being limited thereto.

### [Example 1] Construction of expression vector of human REG protein (REG Iα) and rat Reg protein (Reg I).

Full length of the protein coding region of human REG Iα cDNA (Terazono, K. et al., J. Biol. Chem. 263, 2111-2114 (1998)) was inserted to SnaBI/AvrII site at the downstream of yeast alcohol oxidase promoter of Pichia expression vector pPIC3.5 (Invitrogen) using a linker to construct the expression vector. Full length of the protein coding region of Rat Reg I cDNA (Terazono, K. et al., described above) was also inserted similarly to SnaBI/NotI site of pPIC3.5 using a linker. These two expression vector DNAs were purified by CsCl method, and introduced to competent cells (Pichia GS115 strain) prepared using Pichia Easy Comp Kit (Invitrogen). Cells into which the expression vector has been introduced were selected by the fact that these cells acquire the ability to grow in a medium without histidine. Among these cells, a clone in which the amount of human REG protein or rat Reg protein produced and secreted into the medium becomes maximum when methanol is added was selected.

### [Example 2] Preparation of human REG protein (REG Iα) and rat Reg protein (Reg I)

Pichia (*Pichia pastoris)* producing human REG protein or rat Reg protein described above was precultured at 28∼30°C for 16∼18 hours in the BMGY medium (1% yeast extract, 2% polypeptone, 100 mM potassium phosphate buffer (pH 6.0), 1.34% Yeast Nitrogen Base, 0.00004% biotin, 1% glycerol). Then, it was cultured on a large scale until OD₆₀₀ became 2∼5 in the BMGY medium. The yeast were collected by centrifugation, and resuspended in BMMY medium (1% yeast extract, 2% polypeptone, 100 mM potassium phosphate buffer (pH 6.0), 1.34% Yeast Nitrogen Base, 0.00004% biotin, 0.5% methanol), and cultured at 28∼30°C for 3∼4 days. During the time, methanol was added to a final concentration of 0.5% at intervals of 24 hours. The culture supernatant was collected and acetic acid was added to adjust pH to 3.5. The pH adjusted culture medium was applied to STREAMLINE SP (Pharmacia) equilibrated by 50 mM sodium acetate (pH 3.5), and after washing with 50 mM sodium acetate (pH 3.5), it was eluted with 50 mM sodium acetate (pH 3.5)/0.5 M NaCl. Mass spectrometry was used to confirm that proteins produced were human REG protein or rat Reg protein.

### [Example 3] Effects of addition of REG protein toward rat insulinoma derived cultured cells, the RINm5F cells

REG protein was added to rat insulinoma derived cultured cells, the RINm5F cells (Zenilman, M.E. et al., Gastroenterology 110, 1208-1214 (1996)) and the incorporation of 5'-bromo-2'-deoxyuridine (BrdU) (cell growth activity) was measured. First, 5 x 10⁵ cells/ml of RINm5F cells were seeded onto 96 well plates at 100 µl/well and cultured for 2 days at 37°C. After that, the culture medium was changed to 100 µl/well of the medium described below. As for Human REG Iα, the one described in Example 2 was used.
Medium + 1% FCS
Medium +1% FCS + human REG Iα (1 nM; 0.016 µg/ml)
Medium +1% FCS + human REG Iα (10 nM; 0.16 µg/ml)
Medium +1% FCS + human REG Iα (100 nM; 1.6 µg/ml)
Medium +1% FCS + human REG Iα (1000 nM; 16 µg/ml)

The cells were incubated at 37°C for 24 hours, and then, 10 µl/well of BrdU labeling solution (10 mM BrdU stock solution was diluted with medium to be 100 µM) was added(final concentration 10 µM). After incubation at 37°C for 12 hours, medium was removed and 200 µl/well of FixDenat (Roche Diagnostics) was added. After incubation at room temperature for 15 min, FixDenat solution was removed, and then 100 µl/well of anti-BrdU-POD antibody (1/100 diluted solution of stock solution, Roche Diagnostics) was added. After incubating at room temperature for 60 min, the anti-BrdU-POD antibody solution was removed, and then rinsed three times with 200 µl/well of washing solution (10x washing solution, Roche Diagnostics). 100 µl/well of substrate solution (Roche Diagnostics) was added and incubated at room temperature until a sufficient color development was obtained. Absorbance of each sample at 370 nm was measured using an ELISA reader (reference wavelength: approx. 492 nm).

As a result, REG protein concentration dependent cell growth was observed (Figure 1).

### [Example 4] Assay of the binding activity of Reg protein towards RINm5F cells

First, diluted [¹²⁵I] Reg I solution was prepared as described below. [¹²⁵I] rat Reg I stock solution (50 ng/µl=∼3.33 µM, 8.6 x 10⁵ cpm/µl) was diluted with DMEM to be 1 nM, 333 pM, 100 pM, 33 pM, and 10 pM. Moreover, diluted solution with a 100-fold concentration of non-labeled Reg I stock solution (460 ng/µl= 30.6 µM) was similarly prepared. 3 ml of 4 x 10⁵ cells/ml RINm5F cells were seeded onto 6 well plates and cultured at 37°C for 2 days. After washing with ice cold DMEM, 3 ml of DMEM containing [¹²⁵I] rat Reg I described above was added (final concentration: 10 pM, 33 pM, 100 pM, 333 pM, and 1 nM). In a competitive inhibition experiment, DMEM with a 100-fold amount of non-labeled Reg I was used. After keeping on ice for 2 hours, the cells were washed with DMEM for 3 times, and then lysed by adding 0.5∼1 ml/well of [100 mM Tris-HCl (pH 7.6), 1 mM EDTA, 1% Triton X-100] and [¹²⁵I] radioactivity was counted by γ-counter.

As a result, it was seen that the excessive amount of non-labeled Reg protein inhibited the binding, indicating the existence of a molecule necessary for specific binding on the cell membrane of RINm5F cells (Figure 2).

### [Example 5] Identification and isolation of Reg-binding protein

Rat pancreatic Langerhans' islet expression cDNA library was constructed by the λZAP II vector using poly (A) + RNA of rat pancreatic Langerhans' islets as template. Rat Reg protein prepared in Example 2 was labeled with [¹²⁵I] using Bolton-Hunter reagent, and phage clones binding to Reg protein was selected and isolated by West-Western method from the expressed cDNA library.

Recombination of cDNA into a plasmid vector (pBluescript SK(-), Stratagene) was carried out by an *in vivo* excision method using helper phage from positive phage clones. Nucleotide sequence of cDNA was determined by the dideoxy method. The nucleotide sequence and expected amino acid sequence are shown in SEQ ID NO: 1 and SEQ ID NO: 2, respectively. The Protein estimated from the nucleotide sequence was thought to be a cell membrane protein with a transmembrane domain comprising a hydrophobic amino acid cluster.

### [Example 6] Expression of Reg-binding protein in COS-7 cells

cDNA isolated in Example 5 was integrated into a mammalian cell expression vector comprising a cytomegalovirus promoter (pCI-neo) (Promega) to construct a Reg-binding protein expression vector (pCI-167.1). The vector was introduced into COS-7 cells by electroporation method and expressed transiently. 48 hours after introducing the vector, Reg binding activity was examined by a protocol similar to that described in Example 4.

Specifically, first, [¹²⁵I] rat Reg I stock solution (50 ng/µl =∼3.33 µM, 2.7 x 10⁵ cpm/µl) was diluted to 10 nM using DMEM. In addition, a diluted solution in which non-labeled Reg I stock solution (2250 ng/µl = 150 µM) was added at 100-folds the concentration of [¹²⁵I] Reg I (1 µm), was prepared.

3 ml of 2.5 x 10⁵ cells/ml transfected COS cells were seeded onto 6 well plates and cultured at 37°C for 2 days. After washing with ice-cold DMEM, 3 ml DMEM containing [¹²⁵I] rat Reg I was added (10 nM, final concentration) . In a competitive inhibition experiment, 100-fold amount of non-labeled Reg I coexisted. After keeping on ice for 2 hours and then washing 3 times with DMEM, 1 ml/well of [100 mM Tris-HCl (pH 7.6), 1 mM EDTA, 1% Triton X-100] was added to lyze the cells, and [¹²⁵I] radioactivity was counted by using a γ-counter.

As a result, the binding to [¹²⁵I] labeled Reg protein increased significantly in cDNA-introduced cells, in comparison with the cells to which only the vector was introduced. Moreover, the binding disappeared by the addition of an excessive amount of non-labeled Reg protein (Figure 3). Therefore, it was thought that the protein encoded by the isolated cDNA was a molecule binding to Reg protein on a mammalian cell membrane, and that it can be a receptor molecule which plays a key role in β cell regeneration and proliferation activity of Reg protein.

### [Example 7] Screening of rat pancreatic islet cDNA library

To further isolate cDNA encoding a Reg-binding protein, a rat islet cDNA library (5 x 10⁶ clones) was screened by plaque hybridization using the cDNA fragment obtained in Example 5 as probe, and 8 positive clones were obtained. The 8 clones largely overlapped with each other and had complete nucleotide identity in the overlapping regions. The obtained cDNA sequence encoding rat Reg-binding protein and amino acid sequence of Reg-binding protein encoded by the cDNA are shown in SEQ ID NO: 3 and SEQ ID NO: 4, respectively.

As shown in Figure 4, the cDNA has a 2,760 bp open reading frame encoding a 919 amino acid protein, and the deduced amino acid sequence of the cDNA predicted that the protein is a type II transmembrane domain with a long extracellular domain (868 amino acid residues), a transmembrane domain (residues 29-51) and a short intracellular region at the N-terminus.

### [Example 8] Expression of rat Reg-binding protein

An expression vector for the rat Reg protein cDNA isolated in Example 7 was constructed, and it was transiently expressed in COS-7 cells. The rat Reg binding protein cDNA, into which an oligonucleotide encoding hemagglutinin (HA) nonapeptide-tag (YPYDVPDYA) at the N-terminus was ligated, was inserted into a pCl-neo mammalian expression vector (Promega). This vector was introduced to COS-7 cells by electroporation and expressed. After a 48 h incubation, cells were collected, homogenized, and fractionated as described (S. Takasawa et al., J. Biol. Chem. 268, 26052 (1983); H. Okamoto et al., Meth. Enzymol. 280, 306 (1997)). The protein sample was electrophoresed on a 12.5 % (w/v) SDS-polyacrylamide gel and transferred to immobilon-P (Millipore). Western blot analysis was carried out described as in S. Takasawa et al., J. Biol. Chem. 270, 30257 (1995); H. Okamoto et al., Meth. Enzymol. 280, 306 (1997). Monoclonal antibody against HA was anti-HA 3F10 (Boehringer).

Immunoblot analysis revealed that the protein encoded by the cDNA was expressed predominantly in the cell membrane fraction with an apparent molecular weight of 105 kD (Figure 5A), coinciding with the molecular weight calculated from the presumed amino acid sequence. (104,682).

### [Example 9] Binding of rat Reg-binding protein to Reg protein

The rat Reg-binding protein (also called EXTL3/EXTR1) expression vector constructed in Example 8 or the control vector was introduced into COS-7 cells by electroporation and expressed transiently. CHO cells expressing the Reg receptor stably were isolated as described above. The cells (7.5 x 10⁵ cells) were washed with RPMI1640 (Roswell Park Memorial institute 1640 medium) and incubated on ice in the presence of ¹²⁵I labeled rat Reg protein (50 ng/ml, 1.5 x 10⁵ cpm/ml) with various concentrations of unlabeled rat Reg or human REG protein in RPMI1640 containing 1% fetal calf serum for 2 h. After washing with RPMI1640 three times, cells were solubilized by 1 ml of 100 mM Tris-HCl (pH 7.6), 1 mM EDTA and 1% Triton X-100. The radioactivity of the lysate was-determined by a γ-counter (Cobra, Packard). As a result, rat Reg binding protein expression vector-introduced-COS-7 cells bound to ¹²⁵I-labeled rat Reg protein and the binding was decreased by the addition of unlabeled Reg protein (Figure 6).

A homology search against DNA and protein databases revealed that the cDNA of rat Reg-binding protein (SEQ ID NO: 3) and its deduced amino acid sequence (SEQ ID NO: 4) shows significant homologies to those of multiple exostoses (EXT) family genes, especially to human EXT-like gene 3 (EXTL3)/EXT-related gene 1 (EXTR1) (W. Van Hui et al., Genomics 47, 230(1998); T. Saito et al., Biochem. Biophys. Res. Commun. 243, 61 (1998)) (over 97% amino acid identity), indicating that the cDNA encodes a rat homologue to human EXTL3/EXTR1. The EXTL3/EXTR1 gene has been isolated as a member of the EXT family genes by homology screening, but its physiological function and pathological significance have not yet been clarified. EXTL3/EXTR1 is thought to belong to the EXT family (W. Van Hui et al., Genomics 47, 230 (1998); T. Saito et al., Biochem. Biophys. Res. Commun. 243, 61 (1998)) because it shows homology to EXT2 and EXT1 at their C-terminal regions (52% in C-terminal 262 amino acids with EXT2 and 40% in C-terminal at 247 amino acids with EXT1) (see Figure 1). However, the N-terminal region (residues 1-656) of EXTL3/EXTR1 has no homology to any other members of the EXT family genes . Furthermore, the N-terminal region of EXTL3/EXTR1 contained a transmembrane domain, but the other members of the family lacked this domain, and therefore, were not thought to be cell surface proteins. In addition, the 1.6 kbp cDNA, which was initially isolated in the screening of the rat islet cDNA expression library as a Reg-binding protein, contained only the N-terminal region (amino acid residues 1-332). Therefore, it is reasonable to assume that the N-terminal region contains the Reg binding domain and that the EXT family members other than EXTL3/EXTR1 have no ability to bind to Reg protein.

### [Example 10] Stimulation effect of rat Reg-binding protein (rat Reg receptor) expression cell by Reg protein

The expression vector constructed in Example 8 was introduced into CHO cells and several cell lines overexpressing the receptor protein were established, and 5'-bromo-2'-deoxyuridine (BrdU) incorporation into the cells in response to rat Reg protein stimulation was examined.

The rat receptor expression vector with HA-tag was introduced into CHO cells and RINm5F cells. Cells were cultured in Roswell Park Memorial Institute 1640 medium (RPMI1640) with 10% fetal calf serum (Bio Whittaker, Walkersville, Maryland) and 250 µg/ml neomycin (Gibco) for 2 weeks [S. Takasawa et al., J. Biol. Chem. 273, 2497 (1998)]. Stable transformants expressing high levels of the recombinant protein were screened by immunoblot analysis of HA and isolated. Stable transformants expressing Reg receptor were cultured in RPMI1640 medium with 1% fetal calf serum in the presence of increasing concentrations of rat Reg protein for 24 h. During the last 2 h, BrdU (10 µM) was added to the culture medium and BrdU incorporation was measured using a colorimetric cell proliferation ELISA kit (Boehringer).

The BrdU incorporation of EXTL3/EXTR1 expressing cell lines (both #3 and #22) was significantly increased when incubated with 1-300 nM rat Reg protein (EC₅₀=4.01 nM in line #3 and 1.11 nM in line #22, Figure 7A). The Reg protein concentrations exhibiting growth-stimulating effects on the CHO-cell lines were consistent with those for primary cultured rat islets (T. Watanabe et al., Proc. Natl. Acad. Sci. USA 91, 3589 (1994)), suggesting that the replication of pancreatic β-cells by Reg protein is mediated by the rat homologue to EXTL3/EXTR1.

¹²⁵I-labeled rat Reg protein bound to the CHO cells (K_{d}=4.41 nM) and the binding was displaced by increasing the concentrations of unlabeled rat Reg protein (Kᵢ = 1.61 nM; Figure 7B) (refer to Example 9). The Hill coefficient for rat Reg protein was estimated to be n_{H}=1.18, indicative of interactions with a single, homogenous population of binding sites. In addition, human REG protein (K. Terazono, et al., J. Biol. Chem. 263, 2111 (1988); K. Terazono, T. Watanabe, Y. Yonemura, in Molecular biology of the islets of Langerhans', H. Okamoto, Ed. (Cambridge University Press, Cambridge, 1990), pp. 301-313), which shows a 70% amino acid identity to rat Reg protein, also displaced the binding of radio-labeled rat Reg protein and CHO cells, but the displacement required higher concentrations (Kᵢ=7.41 nM; Figure 7B). These results strongly suggest that EXTL3/EXTR1 is a cell surface Reg receptor that binds to Reg protein and transduces the growth stimulating signals of Reg protein.

### [Example 11] Functional analysis of rat Reg receptor

Reg is recognized as a β-cell growth factor (H. Okamoto, J. Mol. Med. 77, 74 (1999); T. Watanabe et al., Proc. Natl. acad. Sci. USA 91, 3589 (1994) ; D. J. Gross et al., Endocrinology 139, 2369 (1998)). When Reg protein was added to rat insulinoma derived β cell line RINm5F, BrdU incorporation of the cells increased (1.5∼2 fold) showing that it stimulates increase of cell number Reg protein concentration-dependently. It was suggested that since Reg protein concentration stimulating the growth of RINm5F cells coincides with that in rat pancreatic islet primary culture, Reg protein may react through the same receptor in both cells. Next, expression vector constructed in Example 8 was introduced to RINm5F cells to establish several Reg receptor overexpressing cell lines, and using these cell lines the function of Reg protein was examined.

The BrdU incorporation (refer to Example 10 for assay method) of the receptor expressing cell lines (lines #1, #6 and #24) was significantly increased when incubated with 0.3∼300 nM rat Reg protein (Figure 8A).

After a 24 h incubation of the stable transformants expressing Reg receptor in RPMI1640 medium with 1% fetal calf serum in the presence of various concentrations of rat Reg protein, a solution containing WST-1 was added to the medium and cultured further for 30 min and the cleavage of tetrazolium salt 4 [-3- (4-iodophenyl) -2- (4-nitrophenyl) -2H-5-tetrazolio]-1,3-benzene disulfonate (WST-1) by mitochondrial dehydrogenases was measured in viable cells using a Cell Proliferation Reagent WST-1 (Boehringer). The cell number of RINm5F cells were increased in response to the addition of Reg protein (0.3-100 nM), but were reduced when the cells were incubated with high concentrations of Reg protein (Figure 8B).

To evaluate the possibility that a high-concentration of Reg protein induces apoptosis of these cells, this stable transformant expressing Reg receptor was incubated for 24 hr in RPMI1640 medium with 1% fetal calf serum in the presence of increasing concentrations of rat Reg protein. After incubation, apoptosis was detected by the TUNEL method (Y. Gavrieli, Y. Sherman, S. A. Ben-Sasson, J. Cell Biol. 119, 493 (1992)) using an Apoptosis Screening Kit (Wako, Osaka, Japan).

By the apoptosis assay of these cells, it was revealed that the high concentration of Reg protein induced apoptosis of Reg receptor expressing RINm5F cells (Figure 8C). These results indicate that the Reg receptor mediates the proliferation and apoptosis of pancreatic β-cells in response to Reg protein, thereby maintaining a stable β-cell mass.

### [Example 12] Expression assay of Reg receptor mRNA

The expression of the Reg receptor mRNA was examined in various rat tissues and cells by RNase protection assay.

Rat regenerating pancreatic islets were prepared as described before (K. Terazono, et al., J. Biol. Chem. 263, 2111 (1988); K. Terazono, T. Watanabe, Y. Yonemura, in Molecular biology of the islets of Langerhans', H. Okamoto, Ed. (Cambridge University Press, Cambridge, 1990), pp. 301-313; Y. Yonemura et al., Diabetes 33, 401 (1984)). RNAs were isolated from various rat tissues and cell lines. as described before (T. Koguma et al., Biochem. Biophys. Acta 1223, 180 (1994); N. Noguchi et al., J. Biol. Chem.272, 3133 (1997) H. Okamoto et al. Meth. Enzymol. 280, 306 (1997)). The Pst I/Bgt II fragment of rat Reg receptor cDNA was subcloned into the Pst I/Bam HI site of pBluescript SK (-), linearized with Hind III and transcribed in vitro by T3 RNA polymerase using [α-³²P] CTP. The resultant 0.45 kb cRNA was used as a probe. RNase protection assay was performed using an RPA III kit (Ambion) according to instructions.

As shown in Figure 9A, the Reg receptor mRNA was expressed in normal pancreatic islets, regenerating pancreatic islets and RINm5F β-cells. The expression of the Reg receptor was not increased in regenerating pancreatic islets as compared to that in normal pancreatic islets, suggesting that the regeneration and proliferation of pancreatic β-cells that increases β-cell mass is primarily regulated by the expression of Reg protein but not by the expression of the receptor. This hypothesis is consistent with the observations that Reg gene was first identified as a gene specifically expressed in regenerating pancreatic islets (K. Terazono, et al., J. Biol. Chem. 263, 2111 (1988); K. Terazono, T. Watanabe, Y. Yonemura, in Molecular biology of the islets of Langerhans', H. Okamoto, Ed. (Cambridge University Press, Cambridge, 1990), pp. 301-313; K. Terazono et al., Diabetologia 33, 250 (1990)) and that Reg gene expression was also observed in the phase of transient β-cell proliferation such as in pancreatic islets of BB/Wor/Tky rats during the remission phase of diabetes (C. Ishii et al., Endocr. J. 40, 269 (1993)), pancreatic islets of NOD mice during active diabetogenesis (N. J. Baeza et al., Diabetes 45, 67 (1996)) and pancreatic ductal cells (which are thought to be progenitor cells of β-cells), during differentiation and proliferation in a mouse model of autoimmune diabetes (E. Anastasi et al., Eur. J. Endocrinol. 141, 644-52 (1999)). ARIP cells, a pancreatic ductal cell line, which express the Reg receptor (see Figure 9A, lane 6), were also reported to proliferate in a Reg protein-dependent manner (M. E. Zenilman et.al., Gastroenterology 110, 1208 (1996); M. E. Zenilman et al., Pancreas 17, 256 (1998)). The expression of Reg receptor mRNA was also detected in liver, kidney, stomach, small intestine, colon, adrenal gland, pituitary gland and brain, but not in heart (Figure 9B), suggesting the possible involvement of the Reg-Reg receptor signal system as a control mechanism of cell proliferation and apoptosis in a variety of cell types other than pancreatic β-cells. In fact, Reg receptor expressing CHO cell lines proliferated in response to Reg protein (Figure 7A). Furthermore, the CHO cells increased and decreased in number (refer to Example 11 for assay method) depending on the Reg protein concentration (Figure 10).

### Industrial Applicability

The present invention provides a Reg-binding protein (Reg receptor) . Reg protein is a cell growth factor for pancreatic β cells, and it is known that it exerts cell growth activity in epithelial cells, and such, as well. It is thought that the Reg-binding protein has the function of transducing signals required for cell growth by binding with Reg protein in pancreatic β cells, and that pancreatic β cells regenerate through the binding of Reg protein and Reg-binding protein. Therefore, by analyzing the structure of the extracellular domain of Reg-binding protein and searching analogs of the ligand binding to the domain, it is possible to produce "anti-diabetic therapeutic agents" inducing physiological growth of pancreatic β cells. Moreover, since Reg protein does not cause overgrowth of β cells in the pancreas, it is thought that the possibility of causing hypoglycemia, as do overdoses of insulin, does not exist.

## Claims

1. A DNA according to any one of (a) to (i),
(a) a DNA encoding a protein comprising the amino acid sequence of SEQ ID NO: 2,
(b) a DNA comprising the coding sequence of the nucleotide sequence of SEQ ID NO: 1,
(c) a DNA encoding a protein comprising an amino acid sequence in which one or more amino acids of the amino acid sequence of SEQ ID NO: 2 have been substituted, deleted, inserted and/or added, wherein said DNA encodes a protein having the activity of binding to Reg protein,
(d) a DNA hybridizing to a DNA comprising the nucleotide sequence of SEQ ID NO: 1, wherein said DNA encodes a protein having the activity of binding to Reg protein,
(e) a DNA encoding a protein comprising the amino acid sequence of SEQ ID NO: 4,
(f) a DNA comprising the coding region of the nucleotide sequence of SEQ ID NO: 3,
(g) a DNA encoding a protein comprising the amino acid sequence in which one or more amino acids of the amino acid sequence of SEQ ID NO: 4 have been substituted, deleted, inserted and/or added, wherein the DNA encodes a protein having the activity of binding to Reg protein,
(h) a DNA hybridizing to a DNA comprising the nucleotide sequence of SEQ ID NO: 3, wherein said DNA encodes a protein having the activity of binding to Reg protein,
(i) a DNA encoding a partial peptide of a protein comprising the amino acid sequence of SEQ ID NO: 2 or SEQ ID NO: 4.

2. A protein or peptide encoded by the DNA according to claim 1.

3. A vector into which the DNA according to claim 1 has been inserted.

4. A host cell carrying the vector according to claim 3.

5. A method for producing the protein or peptide according to claim 2, wherein said method comprises the following steps of,
(a) culturing the cell according to claim 4, and,
(b) recovering the recombinant protein expressed by the cell from the cultured cell or from the culture supernatant.

6. An antibody against the protein or peptide according to claim 2.

7. Apolynucleotide comprising at least 15 nucleotides, wherein said polynucleotide hybridizes with a DNA selected from the group consisting of SEQ ID NO: 1, SEQ ID NO: 3, and DNA complementary thereto.

8. A method of screening for a compound that binds to the protein or peptide according to claim 2, wherein said method comprises the following steps of,
(a) contacting the protein or peptide with a test sample,
(b) detecting the binding of the test sample to the protein or peptide, and,
(c) selecting a compound that binds to the protein or peptide.

9. A method of screening for a compound that inhibits the binding of Reg protein to the protein or peptide according to claim 2, wherein said method comprises the following steps of,
(a) contacting Reg protein with the protein or peptide according to claim 2 in the presence of a test sample,
(b) detecting the binding of Reg protein to the protein or peptide according to claim 2, and,
(c) selecting a compound that decreases the binding.

10. A compound isolated by the method according to claim 9, wherein said compound inhibits the binding of Reg protein to the protein or peptide according to claim 2.

11. A method of screening for a compound that promotes or inhibits signal transduction caused by an activation of the protein according to claim 2, wherein said method comprises the following steps of,
(a) contacting Reg protein with a cell expressing the protein according to claim 2 on the cell surface, in the presence of a test sample,
(b) detecting a change of the cell in response to the stimulation by Reg protein,
(c) selecting a compound that enhances or suppresses the change of the cell as compared to when detected in the absence of the test sample.

12. The method according to claim 11, wherein said change of the cell detected comprises a change in cell-proliferating activity or DNA-synthesizing activity of the cell.

13. A compound isolated by the method according to claim 11 or 12, wherein said compound promotes or inhibits signal transduction caused by an activation of the protein according to claim 2.

14. A pharmaceutical agent comprising the DNA according to claim 1, the protein or peptide according to claim 2, the vector according to claim 3, the antibody according to claim 6, or the compound according to claim 10 or claim 13.

15. The pharmaceutical agent according to claim 14, wherein said pharmaceutical agent is selected from the group consisting of a Reg-binding agent, a regulator of intracellular signal transduction of cells responding to Reg protein, a cell growth regulator, a DNA synthesis regulator, and an apoptosis regulator.

16. The pharmaceutical agent according to claim 14 or claim 15, wherein said pharmaceutical agent is an anti-diabetic drug.
